# EUROPEAN PATENT SPECIFICATION

(11) **EP 4 441 052 B1**
(45) Date of publication and mention of the grant of the patent: **19.11.2025**
(21) Application number: 22803213.2
(22) Date of filing: 14.10.2022
(51) Int. Cl.: C07D 491/147, C07D 498/14, A61P 3/00, A61P 3/10, A61P 7/02, A61P 9/00, A61P 17/06, A61P 19/08, A61P 25/00, A61P 25/28, A61P 29/00, A61P 31/12, A61P 31/18, A61P 35/00, A61P 35/02, A61P 37/00, A61K 31/5025, A61K 31/5383

(54) **COMPOUNDS**
VERBINDUNGEN
COMPOSÉS

(30) Priority: 01.12.2021 EP 21211778
(43) Date of publication of application: 09.10.2024
(73) Proprietor: Fundación del Sector Público Estatal Centro Nacional de Investigaciones Oncológicas Carlos III (F.S.P. CNIO), 28029 Madrid (ES)
(72) Inventor: FERNÁNDEZ, Joaquin Pastor, 28029 Madrid (ES); BLANCO APARICIO, Carmen, 28029 Madrid (ES); MARTÍNEZ GONZÁLEZ, Sonia, 28029 Madrid (ES); ALBARRÁN SANTIÑO, María Isabel, 28029 Madrid (ES); ÁLVAREZ ESCOBAR, Rosa María, 28029 Madrid (ES); CEBRIÁ GÓMEZ, Antonio, 28029 Madrid (ES); CEBRIÁN MUÑOZ, David Álvaro, 28029 Madrid (ES); GARCÍA GARCÍA, Ana Belén, 28029 Madrid (ES); GÓMEZ DE LA OLIVA, Cristina Ana, 28029 Madrid (ES); GONZÁLEZ CANTALAPIEDRA, Esther, 28029 Madrid (ES); HERNÁNDEZ ENCINAS, Elena, 28029 Madrid (ES); MARTÍN HERNANDO, Jose Ignacio, 28029 Madrid (ES); RAMOS LIMA, Francisco Javier, 28029 Madrid (ES); RIESCO FAGUNDO, Rosario Concepción, 28029 Madrid (ES)
(74) Representative: D Young & Co LLP
(86) International application number: PCT/EP2022/078724
(87) International publication number: WO 2023/099072

(56) References cited:
- WO-A1-2011/080510
- WO-A1-2013/013188

## Description

### Field of the Invention

This invention relates to novel pharmaceutically useful compounds, which compounds are useful as inhibitors of protein or lipid kinases (such as inhibitors of a member of the PIM family of kinases, e.g. PIM-1, PIM-2 or PIM-3). The invention also relates to such compounds for use as medicaments, to such compounds for use *in vitro, in situ* and *in vivo* diagnosis or treatment of mammalian cells (or associated pathological conditions), to pharmaceutical compositions containing them, and to synthetic routes for their production.

### Background to the Invention

The malfunctioning of protein kinases (PKs) is the hallmark of numerous diseases. A large share of the oncogenes and proto-oncogenes involved in human cancers code for PKs. The enhanced activities of PKs are also implicated in many non-malignant diseases, such as benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis. PKs are also implicated in inflammatory conditions and in the multiplication of viruses and parasites. PKs may also play a major role in the pathogenesis and development of neurodegenerative disorders.

For a general reference to PKs malfunctioning or disregulation see, for instance, Current Opinion in Chemical Biology 1999, 3, 459 - 465.

PIM-1 is the proto-oncogene activated by murine leukemia virus (Provirus Integration site for Moloney murine leukemia virus - MoMuLV) that induces T-cell lymphoma [Cuypers, H.T., et. al. Cell, 1984, 37, 141-150].

The expression of the proto-oncogene produces a non-transmembrane serine/threonine kinase of 313 residues, including a kinase domain consisting of 253 amino acid residues. Two isoforms are known through alternative initiation (p44 and p33) [Saris, C.J.M. et al. EMBO J. 1991, 10, 655-664].

PIM-1, PIM-2 and PIM-3 phosphorylate protein substrates that are important in cancer neogenesis and progression. For example, PIM-1 phosphorylates *inter alia* p21, Bad, c-myb, Cdc 25A and elF4B (see e.g. Quian, K. C. et al, J. Biol. Chem. 2005, 280(7), 6130-6137, and references cited therein).

Two PIM-1 homologs have been described [Baytel, D. Biochem. Biophys. Acta 1998, 1442, 274-285; Feldman, J. et al. J. Biol. Chem. 1998, 273, 16535.16543]. PIM-2 and PIM-3 are respectively 58% and 69% identical to PIM-1 at the amino acid level. PIM-1 is mainly expressed in thymus, testis, and cells of the hematopoietic system [Mikkers, H.; Nawijn, M.; Allen, J.; Brouwers, C.; Verhoeven, E.; Jonkers, J.; Berns, Mol. Cell. Biol. 2004, 24, 6104; Bachmann, M.; Moroy, T. Int. J. Biochem. Cell Biol. 2005, 37, 726-730. 6115]. PIM-1 expression is directly induced by STAT (Signal Transducers and Activators of Transcription) transcription factors, and PIM-1 expression is induced by many cytokine signalling pathways such as interleukins (IL), granulocyte-macrophage colony stimulating factor (GM-CSF), α- and γ-interferon, erythropoietin, and prolactin [Wang, Z et al. J. Vet. Sci. 2001, 2, 167-179].

PIM-1 has been implicated in lymphoma development. Induced expression of PIM-1 and the proto-oncogene c-myc synergise to increase the incidence of lymphomagenesis [Breuer, M. et al. Nature 1989, 340, 61-63; van Lohuizen M. et al. Cell, 1991, 65, 737-752]. PIM-1 functions in cytokine signalling pathways and has been shown to play a role in T cell development [Schmidt, T. et al. EMBO J. 1998, 17, 5349-5359; Jacobs, H. et al. JEM 1999, 190, 1059-1068]. Signalling through gp130, a subunit common to receptors of the IL-6 cytokine family, activates the transcription factor STAT3 and can lead to the proliferation of hematopoietic cells [Hirano, T. et al. Oncogene 2000, 19, 2548-2556]. A kinase-active PIM-1 appears to be essential for the gp130-mediated STAT3 proliferation signal. In cooperation with the c-myc PIM-1 can promote STAT3-mediated cell cycle progression and antiapoptosis [Shirogane, T. et al., Immunity, 1999, 11, 709-719]. PIM-1 also appears to be necessary for IL-3-stimulated growth in bone marrow-derived mast cells [Domen, J. et al., Blood, 1993, 82, 1445-1452] and survival of FDCP1 cells after IL-3 withdrawal [Lilly, M. et al., Oncogene, 1999, 18, 4022-4031].

Additionally, control of cell proliferation and survival by PIM-1 may be effected by means of its phosphorylation of the well-established cell cycle regulators cdc25 [Mochizuki, T. et al., J. Biol. Chem. 1999, 274, 18659-18666] and/or p21(Cip1/WAF1) [Wang Z. et al. Biochim. Biophys. Acta 2002, 1593, 45-55] or phosphorylation of heterochromatin protein 1, a molecule involved in chromatin structure and transcriptional regulation [Koike, N. et al, FEBS Lett. 2000, 467, 17-21].

Mice deficient for all three PIM genes showed an impaired response to hematopoietic growth factors and demonstrated that PIM proteins are required for efficient proliferation of peripheral T lymphocytes. In particular, it was shown that PIM function is required for efficient cell cycle induction of T cells in response to synergistic T-cell receptor and IL-2 signalling. A large number of interaction partners and substrates of PIM-1 have been identified, suggesting a pivotal role for PIM-1 in cell cycle control, proliferation, as well as in cell survival.

The oncogenic potential of this kinase has been first demonstrated in E µ PIM-1 transgenic mice in which PIM-1 over-expression is targeted to the B-cell lineage which leads to formation of B-cell tumours [van Lohuizen, M.et al.; Cell 1989, 56, 673-682. Subsequently PIM-1 has been reported to be over-expressed in a number of prostate cancers, erythroleukemias, and several other types of human leukemias [Roh, M.et al.;. Cancer Res. 2003, 63, 8079-8084; Valdman, A. et al; Prostate 2004, 60, 367-371].

For example, chromosomal translocation of PIM-1 leads to overexpression of PIM-1 in diffuse large cell lymphoma. [Akasaka, H.et al.; Cancer Res. 2000, 60, 2335-2341]. Furthermore, a number of missense mutations in PIM-1 have been reported in lymphomas of the nervous system and AIDS-induced non-Hodgkins' lymphomas that probably affect PIM-1 kinase activity or stability [Pasqualucci, L. et al, Nature 2001, 412, 341-346; Montesinos-Rongen, M. et al., Blood 2004, 103, 1869-1875; Gaidano, G. et al., Blood 2003, 102, 1833-184]. Thus, the strong linkage between reported overexpression data and the occurrence of PIM-1 mutations in cancer suggests a dominant role of PIM-1 in tumorigenesis.

Overexpression of PIM1 is associated with poor response to radiotherapy and various forms of chemotherapy, including platinum- and taxane-based therapies. In part, due to drug transporters induced PIM. Moreover, PIM confers resistance to targeted therapy such as PI3K, PI3K/mTOR, AKT, pyruvate dehydrogenase and mTOR inhibitors. On the other hand, PIM pathway has been shown to be activated after TKI treatment such as EGFR, MET and ALK inhibitors. PIM kinases have also been implicated in the resistance to angiogenic treatments with VEGF-A- and VEGFR targeted agents. (Review by Toth RK, Warfel NA. Mol Cancer Ther. 2021 Jan;20(1):3-10.)

Moreover, PIM-1 associated drug resistance through interaction and phosphorylation of various targets in cancer, including breast cancer resistant protein (BRCP), Etk4, P-glycoprotein (P-gp), and FMS-like tyrosine kinase 3 (FLT3), serve it as a promising target in cancer therapy. (Toth RK and Warfel NA, above).

PIM kinases are expressed after TCR and cytokine signaling in CD4+ T cells and have a role in CD4+ T proliferation and full activation and differentiation of naïve CD4+ T cells, as well as in the responses of effector CD4+ T cells, such that PIM inhibition may provide a therapeutic benefit in T cell mediated diseases such as inflammatory bowel disease (IBD). Jackson et al. Cell Immunol. 2012;272(2):200-13; Shen Y, et al. Biomed Res. 2017;28:8267-70.

Indeed, in two different mouse models of inflammatory bowel disease. Pim-1 kinase inhibition attenuates IBD by promoting T-cell differentiation into Foxp3+ regulatory T-cells and by the down-regulation of excessive Th1- and Th17-type immune responses and the inhibition of the overactivation of macrophages. Yue-Ming Shen et al., Dig Dis Sci 2012 Jul;57(7):1822-31; Yueming Shen, Clin Res Hepatol Gastroenterol. 2018 Sep;42(4):382-386.

Moreover, upregulated PIM-1 by IL6 inhibits FOXP3 and consequently suppresses Tregs' suppressive activity in vitro, so targeting PIM-1 can improve the efficacy of antitumor immunotherapies (Z. Li, et al., J. Biol. Chem. 289 (2014) 26872-26881).

Likewise, PIM kinase has shown lymphocyte and NK cells mediated immunomodulatory responses through cytokine production and activation of NF-kB, MYC, and mTOR pathways (Z. Liu, et al. Am. J. Cancer Res. 10 (2020) 4085-4097).

Furthermore, long form of PIM-1 kinase can transmit inflammatory into B cell survival programs by associating with CD180, such that in autoimmune diseases with increased B cell activity PIM inhibition could provide novel therapeutic options, Egli N,. PLoS One 2015; 10: e0142741.

All these data suggest that PIM could be a new potential therapeutic target in the prevention and treatment of human-specific autoimmune diseases.

PIM-1 is upregulated in PBMCs of systemic lupus erythematosus (SLE).patients with active disease and in the kidneys of a lupus mouse model. Mechanistically, PIM-1 activates NFATc1 expression and modulates NLRP3 inflammasome activation via intracellular Ca²⁺ inducing glomerular damage. Moreover, PIM inhibition attenuate renal disease and mortality in a lupus mouse model, positioning PIM as therapeutic target in human Lupus nephritis. Rong Fu et al. Arthritis Rheumatol. 2019 Aug;71(8):1308-1318.

Rheumatoid arthritis (RA) is a systemic autoimmune disease characterized by chronic inflammation of multiple joints. PIM-1 has been found upregulated in RA synovial tissues and is induced in RA fibroblast-like synoviocytes (RA-FLSs) by TNF-α, IL-6 or S100A4. Moreover, PIM inhibition significantly reduced the pro-inflammatory cytokine-induced proliferation migration and MMP production of RA-FLSs *in vitro.* You-Jung Ha, Rheumatology (Oxford) . 2019 Jan 1;58(1):154-164.

On the other hand, PIM kinase inhibition reduces pro-inflammatory effector function of CD4+ T cells from early RA patients that show high expression of PIM1, significantly abrogates progression of an arthritis model that resembles RA and reduces cartilage destruction. Nicola J Maney et al. Arthritis Rheumatol . 2021 Oct;73(10):1820-1830. These results suggest that PIM-1 might represent a potential therapeutic target for RA.

An integrative biology approach that combine network analysis of psoriasis transcriptome data sets with clinically relevant preclinical models allows the elucidation of IL-22 function in psoriasis and the identification of PIM1 as possible therapeutic target for the disease. Gayathri K Perera et al. Sci Transl Med. 2014 Feb 12;6(223):223ra22.

PIM kinases have also been implicated in neurodegenerative disorders such as Alzheimer's disease (AD). In patients, phosphorylated PARS40 correlates with Aβ and tau pathologies as well as cognitive deficits. PIM is the kinase responsible of PARS40 in the brain. In a mouse model of AD inhibition of PIM1 reduces PRAS40 phosphorylation what correlates with a reduction of Aβ and Tau pathology and rescue of cognitive deficits by increasing proteasome function. Ramon Velazquez et al. Mol Neurodegener. 2016 Jul 13;11(1):52.

For efficient viral replication, human immunodeficiency virus type 2 through utilizing viral protein X (Vpx) suppresses the host innate immune system. PIM kinase phosphorylates lentiviral Vpx what regulates its function in downregulating SAMHD1 and produces a reduction in viral infection capacity, meaning PIM kinases could represent potential therapeutic targets for lentiviral infection. Kei Miyakawa et al. Nat Commun. 2019 Apr 23;10(1):1844.

Several other protein kinases have been described in the literature, in which the activity and/or elevated activity of such protein kinases have been implicated in diseases such as cancer, in a similar manner to PIM-1, PIM-2 and PIM-3.

There is a constant need to provide alternative and/or more efficacious inhibitors of protein kinases, and particularly inhibitors of PIM-1, PIM-2 and/or PIM-3. Such modulators are expected to offer alternative and/or improved approaches for the management of medical conditions associated with activity and/or elevated activity of PIM-1, PIM-2 and/or PIM-3 protein kinases.

WO2011/080510 describes generally tricyclic compounds having activity as kinase inhibitors, in particular PIM family kinase inhibitors, and intermediates and processes for their preparation. However, it does not disclose the genus of compounds, in particular the specific compounds, described and claimed herein.

The listing or discussion of an apparently prior-published document in this specification should not necessarily be taken as an acknowledgement that the document is part of the state of the art or is common general knowledge.

### Summary of the Invention

In one aspect of the invention, there is provided a compound of formula I: 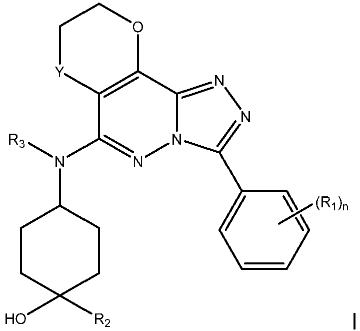 wherein:
Y is selected from the group consisting of C(Rₐ)(R_{b}) and NR_{c};
Rₐ, R_{b} and R_{c} are each independently selected from the group consisting of H and C₁₋₆ alkyl;
n is 1 to 3;
each R₁ is selected from the group consisting of halogen, C₁₋₆ alkyl, C₁₋₆ haloalkyl, C₁₋₆ alkoxy, and C₁₋₆ haloalkoxy;
R₂ is C₁₋₄ alkyl; and
R₃ is selected from the group consisting of H and C₁₋₃ alkyl;
or a pharmaceutically acceptable solvate or salt thereof.

In another aspect of the invention, there is provided a pharmaceutical formulation including a compound of formula I, as defined herein, or a pharmaceutically acceptable solvate or salt thereof, in admixture with a pharmaceutically acceptable adjuvant, diluent or carrier.

In another aspect of the invention, there is provided a compound of formula I as defined herein, or a pharmaceutically acceptable solvate or salt thereof, for use as a medicament.

In another aspect of the invention, there is provided a compound of formula I as defined herein, or a pharmaceutically acceptable solvate or salt thereof, for use in treating a disorder in which a protein or lipid kinase (e.g. a PIM family kinase such as PIM-1, PIM-2 and/or PIM-3) is known to play a role.

In another aspect of the invention, there is provided use of a compound of formula I as defined herein, or a pharmaceutically acceptable solvate or salt thereof, in the manufacture of a medicament for use in treating a disorder in which a protein or lipid kinase (e.g. a PIM family kinase such as PIM-1, PIM-2 and/or PIM-3) is known to play a role.

In another aspect of the invention, there is provided a compound of formula I, as defined herein, for use in a method of treating a disorder in which a protein or lipid kinase (e.g. a PIM family kinase such as PIM-1, PIM-2 and/or PIM-3) is known to play a role in a subject in need thereof, the method comprising administering to the subject a compound of formula I as defined herein, or a pharmaceutically acceptable solvate or salt thereof.

In a further aspect of the present invention, there is provided use of a compound of formula I, as defined herein, in the manufacture of a medicament for the treatment of cancer.

In a further aspect of the present invention, there is provided a compound of formula I, as defined herein, for use in a method of treatment of cancer, which method comprises administration of a therapeutically effective amount of a compound of formula I, as defined herein, to a patient suffering from, or susceptible to, such a condition.

In a further aspect of the present invention, there is provided a compound of formula I, as defined herein, for use in the treatment of cancer.

In another aspect of the invention, there is provided a combination comprising:
(A) a compound of formula I as defined herein, or a pharmaceutically-acceptable solvate or salt thereof; and
(B) another therapeutic agent.

### Brief Description of the Figures

Figure 1 illustrates the anti-proliferative activity of the three Example compounds of the invention, showing the viability of the cells in the A549 (lung cancer) and MiaPaca (pancreatic cancer) cell lines 72 hours after treatment with the three Example compounds and with Comparative Example 1 (Example 19 of WO2011/080510);
Figure 2 compares the fold change of GI₅₀ against these cell lines when treated with the three compounds of the invention and with Comparative Example 1;
Figure 3 illustrates the pharmacokinetics of the three Example compounds of the invention and that of Comparative Example 1 in a Balb/C mouse model, showing the concentration of each compound as a function of time when the compounds are administered at 5 mg/kg intravenously;
Figure 4 illustrates the pharmacokinetics of the three Example compounds of the invention and that of Comparative Example 1 in a Balb/C mouse model, showing the concentration of each compound as a function of time when the compounds are administered at 10 mg/kg orally;
Figure 5 illustrates the anti-proliferative activity of the three Example compounds of the invention, showing the viability of the cells in the MV4:11 (acute myeloid leukaemia), HT-29 (colon cancer), Jeko1 (mantle cell lymphoma) and SKMEL19 (melanoma) cell lines 72 hours after treatment with the three Example compounds and with Comparative Example 1;
Figure 6 compares the fold change of GI50 against these cell lines when treated with the three compounds of the invention and with Comparative Example 1;
Figure 7 shows the tumour volume from 0 to 21 days post-treatment with the compound of Example 1 at doses of 25, 50 and 100 mg/kg compared with control (vehicle);
Figure 8 shows the % change in body weight from 0 to 21 days post-treatment with the compound of Example 1 at doses of 25, 50 and 100 mg/kg compared with control (vehicle);
Figure 9 shows the effect of the compound of Example 1 in inhibiting the generation of Th1 and Th17 responses; and
Figure 10 demonstrates that the compound of Example 1 promotes the generation of iTreg responses.

### Advantages and Surprising Findings

It has been found by the present inventors that the compounds of the present invention exhibit surprisingly improved activity, in particular anti-proliferative activity, against a wide variety of cancer cell lines, compared with the structurally closest compounds as disclosed in WO2011/080510. The compounds therefore have the potential for development as pharmaceuticals for the treatment of a range of indications, in particular cancers, at increased *in vivo* efficacy in patients, compared with the closest compounds as disclosed in WO2011/080510.

It has also been found by the present inventors that the compounds of the present invention exhibit surprisingly improved pharmacokinetic activity, compared with the structurally closest compounds as disclosed in WO2011/080510. This means the compounds would therefore have greater exposure to the site of action *in vivo,* conferring the potential for development as pharmaceuticals having improved efficacy *in vivo* or comparable efficacy at lower doses *in vivo,* compared with the closest compounds as disclosed in WO2011/080510.

### Definitions

"Alkyl" represented by itself means a straight or branched saturated aliphatic radical having a chain of carbon atoms. C_{X} alkyl and C_{X-Y} alkyl are typically used where X and Y indicate the number of carbon atoms in the chain. For example, C₁₋₆ alkyl includes alkyl groups that have a chain of between 1 and 6 carbons (*e.g*., methyl, ethyl, n-propyl, isopropyl, n-butyl, *sec*-butyl, isobutyl, *tert*-butyl, n-pentyl, 2-methylbutyl, neopentyl, n-hexyl, 2-methylpentyl, and 2-ethylbutyl). In some embodiments, alkyl can be a C₁₋₄ alkyl, a C₁₋₃ alkyl or a C₁₋₂ alkyl. Alternatively, alkyl can be a C₁ alkyl, a C₂ alkyl or a C₃ alkyl.

"Alkoxy" means "-O-alkyl", where "alkyl" is as defined above, either in its broadest aspect or a preferred aspect. For example, C₁₋₆ alkoxy includes alkoxy groups that have a chain of between 1 and 6 carbons (*e.g*., methoxy, ethoxy, n-propyloxy, isopropyloxy, n-butyloxy, *sec*-butyloxy, isobutyloxy, *tert*-butyloxy, n-pentyloxy, 2-methylbutyloxy, neopentyloxy, n-hexyloxy, 2-methylpentyloxy, and 2-ethylbutyloxy). In some embodiments, alkoxy can be a C₁₋₄ alkoxy, a C₁₋₃ alkoxy or a C₁₋₂ alkoxy. Alternatively, "alkoxy," can be a C₄ alkoxy, a C₂ alkoxy or a C₃ alkoxy.

"Halogen" means fluorine, chlorine, bromine or iodine.

"Haloalkyl" means alkyl, as defined above, either in its broadest aspect or a preferred aspect, substituted by one or more halogen atoms, as defined above, preferably fluorine or chlorine, more preferably fluorine. The number of halogen atoms is limited only by the number of substitutable positions on the alkyl group, but may be 1 to 5, especially 1, 2 or 3. In one embodiment, "haloalkyl" includes perhaloalkyl (i.e. all of the hydrogen atoms are substituted with halogen atoms, especially fluorine atoms). For example, C₁₋₆ haloalkyl includes alkyl groups that have a chain of between 1 and 6 carbons substituted by one or more halogens (*e.g*., mono-, di or trilfluoromethyl, mono-, di or trichloromethyl, mono-, di, tri-, tetra- or pentafluoroethyl, mono-, di, tri-, tetra- or pentafluoroethyl, 1-, 2- or 3-fluoropropyl, 1, or 2- fluoroisopropyl, 1, 2, 3- or 4-fluoro n-butyl, 1, 2, 3- 4, or 5-fluoro-n-pentyl, 1, 2, 3, 4, 5 or 6-fluoro n-hexyl. In some embodiments, haloalkyl can be a C₁₋₄ haloalkyl, a C₁₋₃ haloalkyl or a C₁₋₂ haloalkyl. Alternatively, haloalkyl can be a C₁ haloalkyl, a C₂ haloalkyl or a C₃ haloalkyl. In some embodiments, haloalkyl can be trilfluoromethyl.

"Haloalkoxy" means alkoxy, as defined above, either in its broadest aspect or a preferred aspect, substituted by one or more halogen atoms, as defined above, preferably fluorine or chlorine, more preferably fluorine. The number of halogen atoms is limited only by the number of substitutable positions on the alkoxy group, but may be 1 to 5, especially 1, 2 or 3. In one embodiment, "haloalkoxy" includes perhaloalkoxy (i.e. all of the hydrogen atoms are substituted with halogen atoms, especially fluorine atoms). For example, C₁₋₆ haloalkoxy includes alkoxy groups that have a chain of between 1 and 6 carbons substituted by one or more halogens (*e.g.*, mono-, di or trifluoromethoxy, mono-, di or trichloromethoxy, mono-, di, tri-, tetra- or pentafluoroethoxy, mono-, di, tri-, tetra- or pentafluoroethoxy, 1-, 2- or 3-fluoro-n-propyloxy, 1, or 2- fluoroisopropyloxy, 1, 2, 3- or 4-fluoro n-butyloxy, 1, 2, 3- 4, or 5-fluoro-n-pentyloxy, or 1, 2, 3, 4, 5 or 6-fluoro-n-hexyloxy. In some embodiments, haloalkoxy can be a C₁₋₄ haloalkoxy, a C₁₋₃ haloalkoxy or a C₁₋₂ haloalkoxy. Alternatively, haloalkoxy can be a C₁ haloalkoxy, a C₂ haloalkoxy or a (C₃)haloalkoxy. In some embodiments, haloalkoxy can be trifluoromethoxy.

### Compounds

The compounds of the invention are compounds of formula I: 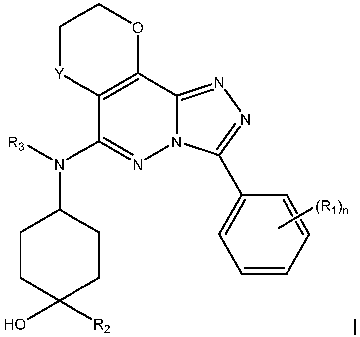 wherein:
Y is selected from the group consisting of C(Rₐ)(R_{b}) and NR_{c};
Rₐ, R_{b} and R_{c} are each independently selected from the group consisting of H and C₁₋₆ alkyl;
n is 1 to 3;
each R₁ is selected from the group consisting of halogen, C₁₋₆ alkyl, C₁₋₆ haloalkyl, C₁₋₆ alkoxy, and C₁₋₆ haloalkoxy;
R₂ is C₁₋₄ alkyl; and
R₃ is selected from the group consisting of H and C₁₋₃ alkyl;
or a pharmaceutically acceptable solvate or salt thereof.

In the compounds of the invention, Y is selected from the group consisting of C(Rₐ)(R_{b}) and NR_{c}.

In one embodiment, Rₐ and R_{b} are each independently selected from the group consisting of H and C₁₋₄ alkyl. In one embodiment, Rₐ and R_{b} are each independently selected from the group consisting of H, methyl and ethyl. In one embodiment, Rₐ and R_{b} are each independently selected from the group consisting of H and methyl. In one embodiment, Rₐ and R_{b} are both H.

In one embodiment, R_{c} is selected from the group consisting of H and C₁₋₄ alkyl. In one embodiment, R_{c} is selected from the group consisting of H, methyl and ethyl. In one embodiment, R_{c} is selected from the group consisting of H and methyl. In one embodiment, R_{c} is H. In one embodiment, R_{c} is methyl.

In one embodiment, Y is selected from the group consisting of CH₂ and N(CH₃). In one embodiment, Y is CH₂. In one embodiment, Y is N(CH₃).

In the compounds of the invention, n is the number of non-hydrogen substituents R₁ on the phenyl ring bearing them. In the compounds of the invention, n is 1, 2 or 3. In one embodiment, n is 1 or 2. In one embodiment, n is 1. In one embodiment, n is 2.

In the compounds of the invention, each R₁ is selected from the group consisting of halogen, C₁₋₆ alkyl, C₁₋₆ haloalkyl, C₁₋₆ alkoxy, and C₁₋₆ haloalkoxy. In one embodiment, each R₁ is selected from the group consisting of halogen, C₁₋₆ haloalkyl, and C₁₋₆ haloalkoxy. In one embodiment, each R₁ is selected from the group consisting of F, Cl, C₁₋₄ haloalkyl, and C₁₋₄ haloalkoxy. In one embodiment, each R₁ is selected from the group consisting of F, Cl, C₁₋₂ haloalkyl, and C₁₋₂ haloalkoxy. In one embodiment, each R₁ is selected from the group consisting of F, mono-, di-or trifluoromethyl, and mono-, di-or trifluoromethoxy. In one embodiment, each R₁ is selected from the group consisting of F, CF₃, and OCF₃.

In one embodiment, R₁ is halogen. In one embodiment, R₁ is F or Cl. In one embodiment, R₁ is F. In one embodiment, R₁ is CH₃. In one embodiment, R₁ is CH₂F. In one embodiment, R₁ is CHF₂. In one embodiment, R₁ is CF₃. In one embodiment, R₁ is OCH₃. In one embodiment, R₁ is OCH₂F. In one embodiment, R₁ is OCHF₂. In one embodiment, R₁ is OCF₃.

In one embodiment, n is 1 and R₁ is OCF₃. In one embodiment, n is 2, one R₁ is F and the other is CF₃.

In the compounds of the invention, R₂ is C₁₋₄ alkyl. In one embodiment, R₂ is methyl, ethyl, or n-propyl. In one embodiment, R₂ is methyl or ethyl. In one embodiment, R₂ is CH₃.

In the compounds of the invention, R₃ is selected from the group consisting of H and C₁₋₃ alkyl. In one embodiment, R₃ is H, methyl, ethyl, or n-propyl. In one embodiment, R₃ is H, methyl or ethyl. In one embodiment, R₃ is H or CH₃. In one embodiment, R₃ is H.

In one embodiment, the OH group on the cyclohexane moiety is in a *cis* relationship to the amino moiety bonding it to the rest of the molecule. In one embodiment, the OH group on the cyclohexane moiety is in a *trans* relationship to the amino moiety bonding it to the rest of the molecule.

In one embodiment, Y is selected from the group consisting of CH₂ and N(CH₃); n is 1 or 2; each R₁ is selected from the group consisting of halogen, C₁₋₆ haloalkyl, and C₁₋₆ haloalkoxy; R₂ is methyl, ethyl, or n-propyl; and R₃ is H, methyl or ethyl.

In one embodiment, Y is selected from the group consisting of CH₂ and N(CH₃); n is 1 or 2; each R₁ is selected from the group consisting of F, Cl, C₁₋₂ haloalkyl, and C₁₋₂ haloalkoxy; R₂ is methyl or ethyl; and R₃ is H or methyl.

In one embodiment, Y is selected from the group consisting of CH₂ and N(CH₃); n is 1 or 2; each R₁ is selected from the group consisting of F, mono-, di-or trifluoromethyl, and mono-, di-or trifluoromethoxy; R₂ is methyl; and R₃ is H.

In one embodiment, there is provided a compound of formula: 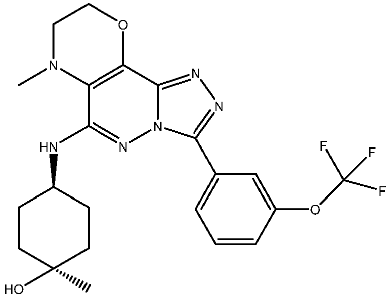 or a pharmaceutically acceptable solvate or salt thereof.

In one embodiment, there is provided a compound of formula: 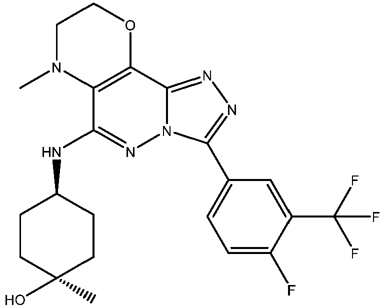 or a pharmaceutically acceptable solvate or salt thereof.

In one embodiment, there is provided a compound of formula: 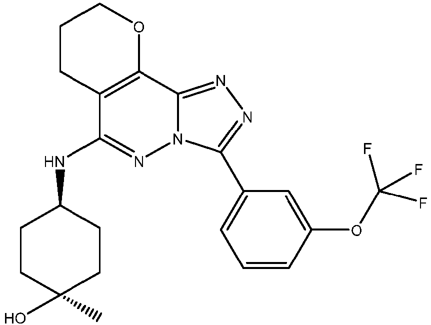 or a pharmaceutically acceptable solvate or salt thereof.

### General Methods

The compounds of formula I may be synthesised according to Scheme 1.

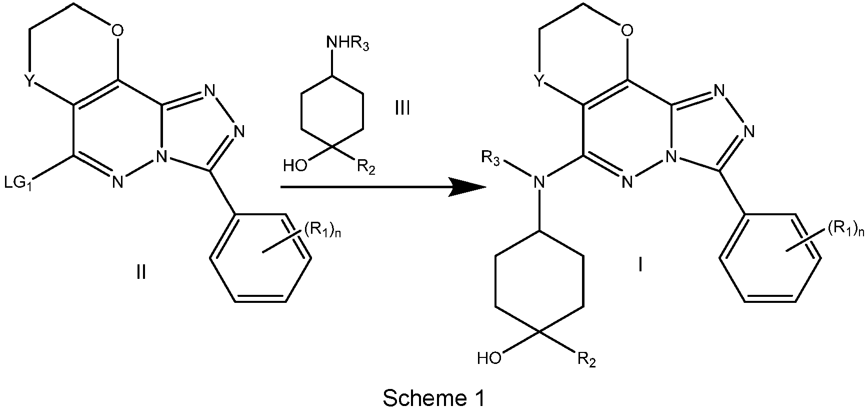

In Scheme 1, Y, n, R₁, R₂ and R₃ are as defined above, and LG₁ represents a suitable leaving group, such as iodo, bromo, chloro or a sulfonate group (e.g. -OS(O)₂CF₃, -OS(O)₂CH₃ or -OS(O)₂-p-tolyl).

According to a further aspect of the invention, there is provided a process for the preparation of a compound of formula I, as defined herein, which process comprises reaction of a compound of formula II: 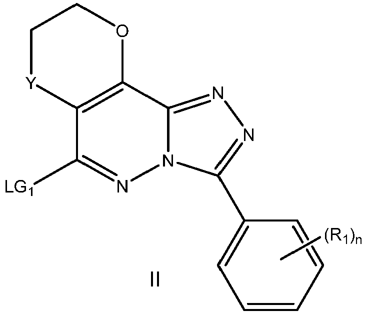 wherein LG₁ represents a suitable leaving group, such as iodo, bromo, chloro or a sulfonate group (e.g. -OS(O)₂CF₃, -OS(O)₂CH₃ or -OS(O)₂-p-tolyl), and R₁ and Y are as defined above, with a compound of formula III: 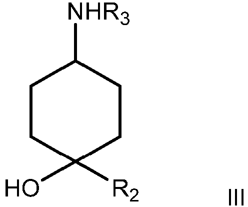 wherein R₂ and R₃ are as defined above.

This process may be carried out under any conditions suitable for a displacement of a leaving group on an aromatic compound by an amino group.

In one embodiment, the reaction is carried out in the presence of an appropriate metal catalyst (or a salt or complex thereof). There is no particular restriction on the nature of the catalyst provided it is capable of catalysing the displacement of a leaving group on an aromatic compound by an amino group. Examples of suitable catalysts include Cu, Cu(OAc)₂, Cul (or Cul/diamine complex), copper tris(triphenylphosphine) bromide, Pd(OAc)₂, tris(dibenzylideneacetone)-dipalladium(0) (Pd₂(dba)₃) or NiCl₂ and an optional additive such as triphenylphosphine (Ph₃P), 2,2'-bis(diphenylphosphino)-1,1'-binaphthyl, (9,9-dimethyl-9H-xanthene-4,5-diyl)bis(diphenylphosphane) (xantphos), NaI or an appropriate crown ether such as 18-crown-6-benzene. Xantphos is preferred.

In one embodiment, the reaction is carried out in the presence of an appropriate base. The base is not particularly restricted provided it is capable of acting as a base. Examples of suitable bases include such as NaH, triethylamine, pyridine, *N,N'-*dimethylethylenediamine, Na₂CO₃, K₂CO₃, K₃PO₄, Cs₂CO₃, sodium or potassium *tert-*butoxide (or a mixture thereof), optionally in the presence of 4Å molecular sieves. Sodium tert-butoxide is preferred.

In one embodiment, the reaction is carried out in a suitable solvent. The solvent is not particularly restricted provided it is inert to the reaction and is capable of dissolving the reactants to at least some extent. Examples of suitable solvents include dichloromethane, dioxane, toluene, ethanol, isopropanol, dimethylformamide, ethylene glycol, ethylene glycol dimethyl ether, water, dimethylsulfoxide, acetonitrile, dimethylacetamide, *N*-methylpyrrolidinone, tetrahydrofuran or a mixture thereof. Dioxane is preferred.

In alternative embodiments, the reaction may be carried out under microwave irradiation reaction conditions or, alternatively, the reaction may be performed in the absence of other reagents such as catalyst, base and even solvent.

Compounds of formula II wherein Y is NR_{c} are disclosed in WO2011/080510. By way of example, a compound of formula II wherein LG₁ is chloro, Y is N-CH₃ and the substituent R₁ on the phenyl moiety is *meta*-OCF₃ is disclosed as Intermediate 18 in WO2011/080510.

Compounds of formula III are commercially available.

Compounds of formula II wherein Y is C(Rₐ)(R_{b}) (referred to below as compounds of formula Ila) may be synthesised according to Scheme 2.

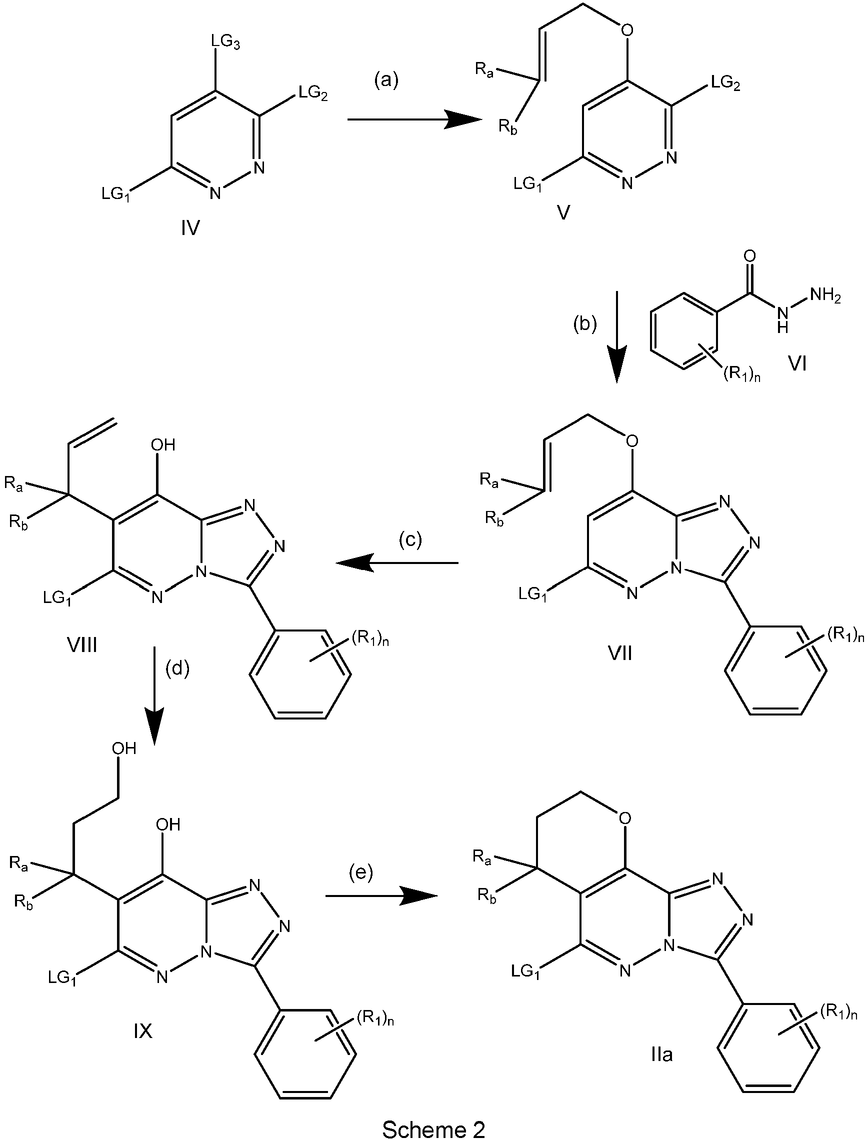

In Scheme 2, n, R₁, Rₐ, and R_{b} are as defined above, and LG₁, LG₂ and LG₃ each independently represents a suitable leaving group, such as iodo, bromo, chloro or a sulfonate group (e.g. -OS(O)₂CF₃, -OS(O)₂CH₃ or -OS(O)₂-p-tolyl).

Step (a) comprises reaction of a compound of formula IV with an allylic alcohol or alkoxide in the presence of a base with displacement of the leaving group LG₃ by an allyloxy group to form a compound of formula V. This process may be carried out under any conditions suitable for a displacement of a leaving group on an aromatic compound by an alcohol or alkoxide.

The reaction is carried out in the presence of an appropriate base. The base is not particularly restricted provided it is capable of acting as a base. Examples of suitable bases include NaH, triethylamine, pyridine, *N*,*N*'-dimethylethylenediamine, Na₂CO₃, K₂CO₃, K₃PO₄, Cs₂CO₃, sodium or potassium tert-butoxide (or a mixture thereof), optionally in the presence of 4Å molecular sieves. Cs₂CO₃ is preferred.

In one embodiment, the reaction is carried out in a suitable solvent. The solvent is not particularly restricted provided it is inert to the reaction and is capable of dissolving the reactants to at least some extent. Examples of suitable solvents include dichloromethane, dioxane, toluene, ethanol, isopropanol, dimethylformamide, ethylene glycol, ethylene glycol dimethyl ether, water, dimethylsulfoxide, acetonitrile, dimethylacetamide, N-methylpyrrolidinone, tetrahydrofuran or a mixture thereof. Acetonitrile is preferred.

Step (b) comprises reaction of a compound of formula V with a benzoic acid hydrazide of formula VI and ring closure to form a compound of formula VII. This process may be carried out under any conditions suitable for a displacement of a leaving group on an aromatic compound by an alcohol or alkoxide.

Certain compounds of formula VI are commercially available. Other compounds of formula VI can be made by reacting the corresponding benzoic acid or acid halide with hydrazine.

In one embodiment, the reaction is carried out in the presence of an appropriate acid. The acid is not particularly restricted provided it is capable of acting as an acid. Examples of suitable acids include strong mineral acids such as hydrochloric acid, nitric acid and sulphuric acid, and sulfonic acids such as methanesulfonic acid, p-toluenesulfonic acid and trifluoromethanesulfonic acid. p-Toluenesulfonic acid is preferred.

In one embodiment, the reaction is carried out in a suitable solvent. The solvent is not particularly restricted provided it is inert to the reaction and is capable of dissolving the reactants to at least some extent. Examples of suitable solvents include dichloromethane, dioxane, toluene, ethanol, isopropanol, dimethylformamide, ethylene glycol, ethylene glycol dimethyl ether, water, dimethyl sulfoxide, acetonitrile, dimethylacetamide, *N*-methylpyrrolidinone, tetrahydrofuran or a mixture thereof. Dioxane is preferred.

Step (c) comprises an allylic rearrangement of a compound of formula VII to form a compound of formula VIII. This process may be carried out under any conditions suitable for carrying out such a rearrangement.

In one embodiment, the reaction is carried out under microwave irradiation reaction conditions. In one embodiment, the reaction is carried out simply by heating the compound of formula VII. Typical temperatures range from 50 to 200°C, preferably 130 to 180°C.

In one embodiment, the reaction is carried out in a suitable solvent. The solvent is not particularly restricted provided it is inert to the reaction and is capable of dissolving the reactants to at least some extent. Examples of suitable solvents include dichloromethane, 1,2-dichloroethane, dioxane, toluene, ethanol, isopropanol, dimethylformamide, ethylene glycol, ethylene glycol dimethyl ether, water, dimethylsulfoxide, acetonitrile, dimethylacetamide, *N*-methylpyrrolidinone, tetrahydrofuran or a mixture thereof. 1,2-dichloroethane is preferred.

Step (d) comprises hydration of the alkene of formula VIII to form a diol compound of formula IX. This process may be carried out under any conditions capable of effecting anti-Markovnikov addition of a water molecule to an alkene.

In one embodiment, the reaction is carried out by hydroboration of the alkene of formula VIII followed by oxidation of the resulting alkylborane intermediate to form the alkene of formula IX.

Examples of suitable hydroborating reagents include borane (optionally in a complex with an ether, such as tetrahydrofuran, or a thioether, such as dimethylsulfide), 9-borabicyclo(3.3.1)nonane (9-BBN), catecholborane, and disiamylborane. Borane-dimethylsulfide is preferred.

The oxidising agent is not particularly restricted provided it is capable of oxidising the alkylborane while being inert to other groups on the molecule. Examples of suitable oxidising agents include compounds containing a peroxide group, such as hydrogen peroxide, peracids and salts thereof. Sodium perborate is preferred.

In one embodiment, the reaction is carried out in a suitable solvent. The solvent is not particularly restricted provided it is inert to the reaction and is capable of dissolving the reactants to at least some extent. Examples of suitable solvents include dichloromethane, dioxane, toluene, ethanol, isopropanol, dimethylformamide, ethylene glycol, ethylene glycol dimethyl ether, water, dimethylsulfoxide, acetonitrile, dimethylacetamide, *N*-methylpyrrolidinone, tetrahydrofuran or a mixture thereof. Tetrahydrofuran is preferred.

Step (e) comprises dehydration and ring closure of a compound of formula IX to form a compound of formula Ila. This process may be carried out under any conditions suitable for carrying out such a dehydration and ring closure. For example, the dehydration can be carried out by treating the diol with an acid or with a base.

In one embodiment, the process is carried out by reacting the diol compound of formula IX with Vilsmeier's reagent (1-chloro-N,N-dimethyliminium chloride).

In one embodiment, the reaction is carried out in the presence of an appropriate acid. The acid is not particularly restricted provided it is capable of acting as an acid. Examples of suitable acids include strong mineral acids such as hydrochloric acid, nitric acid and sulphuric acid, and sulfonic acids such as methanesulfonic acid, p-toluenesulfonic acid and trifluoromethanesulfonic acid. p-Toluenesulfonic acid is preferred.

In one embodiment, the reaction is carried out in the presence of an appropriate base. The base is not particularly restricted provided it is capable of acting as a base. Examples of suitable bases include such as NaH, triethylamine, pyridine, *N,N*'-dimethylethylenediamine, Na₂CO₃, K₂CO₃, K₃PO₄, Cs₂CO₃, sodium or potassium *tert-*butoxide (or a mixture thereof), optionally in the presence of 4Å molecular sieves. Triethylamine is preferred.

In one embodiment, the reaction is carried out in a suitable solvent. The solvent is not particularly restricted provided it is inert to the reaction and is capable of dissolving the reactants to at least some extent. Examples of suitable solvents include dichloromethane, 1,2-dichloroethane, dioxane, toluene, ethanol, isopropanol, dimethylformamide, ethylene glycol, ethylene glycol dimethyl ether, water, dimethylsulfoxide, acetonitrile, dimethylacetamide, *N*-methylpyrrolidinone, tetrahydrofuran or a mixture thereof. 1,2-dichloroethane is preferred.

### Salts, Solvates, Polymorphs, Enantiomers, Isotopically Labelled Derivatives

Pharmaceutically acceptable salts of the compounds of formula I include the acid addition and base salts thereof.

Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

For a review on suitable salts, see Handbook of Pharmaceutical Salts: Properties, Selection, and Use by Stahl and Wermuth (Wiley-VCH, 2002).

Pharmaceutically acceptable salts of compounds of formula I may be prepared by one or more of three methods:
(i) by reacting the compound of formula I with the desired acid or base;
(ii) by removing an acid- or base-labile protecting group from a suitable precursor of the compound of formula (I) or by ring-opening a suitable cyclic precursor, for example, a lactone or lactam, using the desired acid or base; or
(iii) by converting one salt of the compound of formula (I) to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of the invention may exist in a continuum of solid states ranging from fully amorphous to fully crystalline. The term 'amorphous' refers to a state in which the material lacks long range order at the molecular level and, depending upon temperature, may exhibit the physical properties of a solid or a liquid. Typically such materials do not give distinctive X-ray diffraction patterns and, while exhibiting the properties of a solid, are more formally described as a liquid. Upon heating, a change from solid to liquid properties occurs which is characterised by a change of state, typically second order ('glass transition').

The term 'crystalline' refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterised by a phase change, typically first order ('melting point').

The compounds of the invention may also exist in unsolvated and solvated forms. The term 'solvate' is used herein to describe a molecular complex comprising the compound of the invention and one or more pharmaceutically acceptable solvent molecules, for example, ethanol. The term 'hydrate' is employed when said solvent is water.

A currently accepted classification system for organic hydrates is one that defines isolated site, channel, or metal-ion coordinated hydrates - see Polymorphism in Pharmaceutical Solids by K. R. Morris (Ed. H. G. Brittain, Marcel Dekker, 1995). Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion.

When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

In this specification all references to compounds of formula I include references to pharmaceutically acceptable salts and solvates thereof.

The compounds of the invention include compounds of formula (I) as hereinbefore defined, including all polymorphs and crystal habits thereof, stereoisomers thereof (including optical, geometric and tautomeric isomers) as hereinafter defined and isotopically-labeled compounds of formula (I).

Compounds of formula I containing one or more asymmetric carbon atoms can exist as two or more stereoisomers. Where structural isomers are interconvertible via a low energy barrier, tautomeric isomerism ('tautomerism') can occur. This can take the form of proton tautomerism in compounds of formula I containing, for example, an imino, keto, or oxime group, or so-called valence tautomerism in compounds which contain an aromatic moiety. It follows that a single compound may exhibit more than one type of isomerism.

Included within the scope of the present invention are all stereoisomers, geometric isomers and tautomeric forms of the compounds of formula (I), including compounds exhibiting more than one type of isomerism, and mixtures of one or more thereof. Also included are acid addition or base salts wherein the counterion is optically active, for example, d-lactate or /- lysine, or racemic, for example, d--tartrate or d/arginine.

*Cis*/*trans* isomers may be separated by conventional techniques well known to those skilled in the art, for example, chromatography and fractional crystallisation. Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of formula (I) contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. Concentration of the eluate affords the enriched mixture.

When any racemate crystallises, crystals of two different types are possible. The first type is the racemic compound (true racemate) referred to above wherein one homogeneous form of crystal is produced containing both enantiomers in equimolar amounts. The second type is the racemic mixture or conglomerate wherein two forms of crystal are produced in equimolar amounts each comprising a single enantiomer.

While both of the crystal forms present in a racemic mixture have identical physical properties, they may have different physical properties compared to the true racemate. Racemic mixtures may be separated by conventional techniques known to those skilled in the art - see, for example, Stereochemistry of Organic Compounds by E. L. Eliel and S. H. Wilen (Wiley, 1994).

The present invention includes all pharmaceutically acceptable isotopically-labelled compounds of formula (I) wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, and oxygen, such as ¹⁵O, ¹⁷O and ¹⁸O.

Certain isotopically-labelled compounds of formula I, for example, those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection.

Substitution with heavier isotopes such as deuterium, i.e. ²H, may afford certain therapeutic advantages resulting from greater metabolic stability, for example, increased in vivo half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labelled compounds of formula I can generally be prepared by conventional techniques known to those skilled in the art or by processes analogous to those described in the accompanying Examples and Intermediates using an appropriate isotopically-labelled reagent in place of the non-labelled reagent previously employed.

Pharmaceutically acceptable solvates in accordance with the invention include those wherein the solvent of crystallization may be isotopically substituted, e.g. D₂O, d₆-acetone, d₆-DMS.

### Pharmaceutical Compositions

Pharmaceutical compositions suitable for the delivery of compounds of the present invention and methods for their preparation will be readily apparent to those skilled in the art. Such compositions and methods for their preparation may be found, for example, in Remington's Pharmaceutical Sciences. 19th Edition (Mack Publishing Company, 1995).

The compounds of the invention may be administered orally. Oral administration may involve swallowing, so that the compound enters the gastrointestinal tract, and/or buccal, lingual, or sublingual administration by which the compound enters the blood stream directly from the mouth.

Formulations suitable for oral administration include solid, semi-solid and liquid systems such as tablets; soft or hard capsules containing multi- or nano-particulates, liquids, or powders; lozenges (including liquid-filled); chews; gels; fast dispersing dosage forms; films; ovules; sprays; and buccal/mucoadhesive patches.

Liquid formulations include suspensions, solutions, syrups and elixirs. Such formulations may be employed as fillers in soft or hard capsules (made, for example, from gelatin or hydroxypropylmethylcellulose) and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, H (6), 981- 986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkylsubstituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant. Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets. Vol. 1, by H. Lieberman and L. Lachman (Marcel Dekker, New York, 1980).

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Pharmaceutical Technology On-line. 25(2), 1-14, by Verma et al (2001). The use of chewing gum to achieve controlled release is described in WO 00/35298.

The compounds of the invention may also be administered parenterally, i.e. directly into the blood stream, into muscle, or into an internal organ. Suitable means for parenteral administration include intravenous, intraarterial, intraperitoneal, intrathecal, intraventricular, intraurethral, intrasternal, intracranial, intramuscular, intrasynovial and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula I used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a suspension or as a solid, semi-solid, or thixotropic liquid for administration as an implanted depot providing modified release of the active compound. Examples of such formulations include drug-coated stents and semi-solids and suspensions comprising drug- loaded poly([alpha]7-lactic-coglycolic)acid (PGLA) microspheres.

The compounds of the invention may also be administered topically, (intra)dermally, or transdermal to the skin or mucosa. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J. Pharm. Sci., 88 (10), 955-958, by Finnin and Morgan (October 1999).

Other means of topical administration include delivery by electroporation, iontophoresis, phonophoresis, sonophoresis and microneedle or needle-free (e.g. Powderject^{™}, Bioject^{™}, etc.) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler, as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane, or as nasal drops. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as /-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 20mg of the compound of the invention per actuation and the actuation volume may vary from 1 µl to 100µl. A typical formulation may comprise a compound of formula I, propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted and programmed release.

The compounds of the invention may be administered rectally or vaginally, for example, in the form of a suppository, pessary, or enema. Cocoa butter is a traditional suppository base, but various alternatives may be used as appropriate. Formulations for rectal/vaginal administration may be formulated to be immediate and/or modified release. Modified release formulations include delayed-, sustained-, pulsed-, controlled-, targeted and programmed release.

The compounds of the invention may also be administered directly to the eye or ear, typically in the form of drops of a micronised suspension or solution in isotonic, pH-adjusted, sterile saline. Other formulations suitable for ocular and aural administration include ointments, gels, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, poly(vinyl alcohol), hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins, examples of which may be found in International Patent Publication Nos.

WO 91/11172, WO 94/02518 and WO 98/55148.

### Dosage

For administration to human patients, the total daily dose of the compounds of the invention is typically in the range 1 mg to 5000 mg, preferably 10 mg to 2000 mg, more preferably 50 mg to 1500 mg, and still more preferably 100 to 500 mg depending, of course, on the mode of administration. The total daily dose may be administered in single or divided doses and may, at the physician's discretion, fall outside of the typical range given herein.

These dosages are based on an average human subject having a weight of about 60kg to 70kg. The physician will readily be able to determine doses for subjects whose weight falls outside this range, such as infants and the elderly.

For the avoidance of doubt, references herein to "treatment" include references to curative, palliative and prophylactic treatment.

### Combinations

According to another aspect of the invention, there is provided a combination comprising:
(A) a compound of formula I as defined herein, or a pharmaceutically-acceptable solvate or salt thereof; and
(B) another therapeutic agent.

Compounds of the invention may be combined with other therapeutic agents, the other therapeutic agent being active against the disease or condition which is intended to be treated.

In one embodiment, compounds of the invention may also be combined with other therapeutic agents that are inhibitors of protein or lipid kinases (e.g. a PIM family kinase such as PIM-1, PIM-2 and/or PIM-3) and/or useful in the treatment of a cancer and/or a proliferative disease. Compounds of the invention may also be combined with other therapies (e.g. radiation).

For instance, compounds of the invention may be combined with one or more treatments independently selected from surgery, one or more anti-cancer/antineoplastic/anti-tumoral agent, one or more hormone therapies, one or more antibodies, one or more immunotherapies, radioactive iodine therapy, and radiation.

More specifically, compounds of the invention may be combined with an agent that modulates the Ras/Raf/Mek pathway (e.g. an inhibitor of MEK), the Jak/Stat pathway (e.g. an inhibitor of Jak), the PI3K/Akt pathway (e.g. an inhibitor of Akt, PI3K, mTOR), the DNA damage response mechanism (e.g. an inhibitor of ATM or ATR) or the stress signaling pathway (an inhibitor of p38 or NF-*K*B).

For instance, compounds of the invention may be combined with:
(i) a targeted kinase inhibitor;
(ii) a tyrosine kinase inhibitor, such as acalabrutinib, adavosertib, afatinib, afibercept, axitinib, bafetinib, bosutinib, crizotinib, dasatinib, erlotinib, evobrutinib, fenebrutinib, gefitinib, ibrutinib, imatinib, lapatinib, larotrectinib, nilotinib, pazopanib, ponatinib, pirtobrutinib, regorafenib, ruxolitinib, sorafenib, spebrutinib, sunitinib, tepotinib, tirabrutinib, tolebrutinib, vandenatinib, or zanubrutinib;
(iii) an Akt or PI3-K inhibitor, such as pictilisib, dactolisib (BEZ235), copanlisib, duvelisib, umbralisib, alpelisib, zandelisib, parcaclisib or buparlisib;
(iv) an Flt-3 inhibitor;
(v) a BCL-2 inhibitor, such as oblimersen, navitoclax or venetoclax;
(vi) a therapeutic monoclonal antibody, such as adalimumab, alemtuzumab, atezolizumab, AMP-224, AMP-514, AUNP12, avelumab, bevacizumab, BMS-986169, CA-170, camrelizumab, certolizumab pegol, cemiplimab, cetuximab, cosibelimab, denosumab, dostarlimab, durvlalumab, gemtuzamab ozogamicin, golimumab, ibritomumab tiuexetan, ilipimumab, infliximab, nivolumab, ofatunumab, panitumumab, pembrolizumab, pertuzumab, rituximab, sintilimab, spartalizumab, tiselizumab, toripalimab, tositozumab or trastuzumab;
(vii) a MEK inhibitor, such as cobimetinib, selumetinib, mirdametinib (PD-0325901), trametinib, binimetinib or TAK-733;
(vii) a BRaf inhibitor, such as encorafenib, dabrafenib, vemurafenib or GDC-0879;
(viii) an anthracycline, such as daunorubicin, doxorubicin, epirubicin, idarubicin, mitoxantrone or valrubicin;
(ix) a taxane, such as cabazitaxel, paclitaxel or docetaxel;
(x) a platin, such as carboplatin, cisplatin, nedaplatin or oxaliplatin;
(xi) a nucleotide analog, such as azacitidine, capecitabine, carmofur, cladribine, clofarabine, cytarabine, decitabine, floxuridine, 5-fluorouracil, gemcitabine, mercaptopurine, nelarabine, pentostatin, tegafur or tioguanine;
(xii) an alkylating agent, such as bendamustine, busulfan, carmustine, clorambucil, clormethine, cyclophosphamide, dacarbazine, folemustine, ifosfamide, lomustine, melphalan, streptozotocin or temozolomide;
(xiii) a hormone therapeutic agent, such as an estrogen receptor antagonist e.g. tamoxifen;
(xiv) an anti-tumour compound that has potential radiosensitising and/or chemosensitising effects, such as chloroquine;
(xv) an mTOR inhibitor, such as sirolimus (rapamycin), everolimus, ridaforolimus or temsirolimus;
(xvi) a JAK inhibitor, such as ruxolitinib, itacitinib, tofacitinib, oclacitinib, barictinib, peficitinib, fedratinib, upadacitinib, filgotinib, deglocitinib, cerdulatinib, gandotinib, lestaurtinib, momelotinib, pacritinib, abrocitiniib, deucravacitinib, CHZ868 or cucurbitacin;
(xvii) a cyclin dependent kinase inhibitor, e.g. a CDK6 or CDK4 inhibitor, such as palbociclib (PD-0332991), ribociclib, abemaciclib or trilaciclib;
(xviii) an agent that modulates the DNA damage response mechanism and/or the stress signaling pathway, e.g. an inhibitor of ATM or ATR, an inhibitor of p38 and/or NF-KB;
(xix) an MCL-1 inhibitor, such as omacetaxine mepesuccinate or seliclilib;
(xx) a KRAS inhibitor, such as sotorasib (AMG 510), adagrasib (MRTX 849) or ARS-3248;
(xxi) an antiandrogen, such as cyproterone acetate, flutinamide, nilutamide, bicalutamide or enzalutamide;
(xxii) a TNF inhibitor, including some of the monoclonal antibodies listed above, as well as etenercept, pentoxifylline and bupropion; or
(xxiii) a steroid, such as prednisolone, methylprednisolone, dexamethasone or hydrocortisone.

According to a further aspect of the invention, there is provided a combination product comprising:
(A) a compound of the invention, as hereinbefore defined; and
(B) another therapeutic agent that is useful in the treatment of cancer and/or a proliferative disease,
wherein each of components (A) and (B) is formulated in admixture with a pharmaceutically-acceptable adjuvant, diluent or carrier.

Such combination products provide for the administration of a compound of the invention in conjunction with the other therapeutic agent, and may thus be presented either as separate formulations, wherein at least one of those formulations comprises a compound of the invention, and at least one comprises the other therapeutic agent, or may be presented (i.e. formulated) as a combined preparation (i.e. presented as a single formulation including a compound of the invention and the other therapeutic agent).

Thus, there is further provided:
(1) a pharmaceutical formulation including a compound of the invention, as hereinbefore defined, another therapeutic agent that is useful in the treatment of cancer and/or a proliferative disease, and a pharmaceutically-acceptable adjuvant, diluent or carrier; and
(2) a kit of parts comprising components:
   (a) a pharmaceutical formulation including a compound of the invention, as hereinbefore defined, in admixture with a pharmaceutically-acceptable adjuvant, diluent or carrier; and
   (b) a pharmaceutical formulation including another therapeutic agent that is useful in the treatment of cancer and/or a proliferative disease in admixture with a pharmaceutically-acceptable adjuvant, diluent or carrier,
   which components (a) and (b) are each provided in a form that is suitable for administration in conjunction with the other.

In a particularly preferred aspect of the invention, compounds of the invention may be combined with other therapeutic agents (e.g. chemotherapeutic agents) for use as medicaments (e.g. for use in the treatment of a disease or condition as mentioned herein, such as one in which the inhibition of growth of cancer cells are required and/or desired e.g. for treating hyperproliferative disorders such as cancer (e.g. specific cancers that may be mentioned herein, e.g. in the examples) in mammals, especially humans). Such active ingredients in combinations may act in synergy.

The invention further provides a process for the preparation of a combination product as hereinbefore defined, which process comprises bringing into association a compound of the invention, as hereinbefore defined, or a pharmaceutically acceptable ester, amide, solvate or salt thereof with the other therapeutic agent that is useful in the treatment of cancer and/or a proliferative disease, and at least one pharmaceutically-acceptable adjuvant, diluent or carrier.

By "bringing into association", we mean that the two components are rendered suitable for administration in conjunction with each other.

Thus, in relation to the process for the preparation of a kit of parts as hereinbefore defined, by bringing the two components "into association with" each other, we include that the two components of the kit of parts may be:
(i) provided as separate formulations (i.e. independently of one another), which are subsequently brought together for use in conjunction with each other in combination therapy; or
(ii) packaged and presented together as separate components of a "combination pack" for use in conjunction with each other in combination therapy.

The compounds of the invention may also be combined with other therapeutic agents useful in the treatment of diseases other than cancer. By way of example, in the treatment of autoimmune diseases or inflammatory diseases, the compounds of the invention may also be combined with other therapeutic agents useful in the treatment of such diseases.

According to a further aspect of the invention, there is provided a combination product comprising:
(A) a compound of the invention, as hereinbefore defined; and
(B) another therapeutic agent that is useful in the treatment of an inflammatory disease and/or an autoimmune disease,
wherein each of components (A) and (B) is formulated in admixture with a pharmaceutically-acceptable adjuvant, diluent or carrier.

Thus, there is further provided:
(1) a pharmaceutical formulation including a compound of the invention, as hereinbefore defined, another therapeutic agent that is useful in the treatment of an inflammatory disease and/or an autoimmune disease, and a pharmaceutically-acceptable adjuvant, diluent or carrier; and
(2) a kit of parts comprising components:
   (a) a pharmaceutical formulation including a compound of the invention, as hereinbefore defined, in admixture with a pharmaceutically-acceptable adjuvant, diluent or carrier; and
   (b) a pharmaceutical formulation including another therapeutic agent that is useful in the treatment of an inflammatory disease and/or an autoimmune disease in admixture with a pharmaceutically-acceptable adjuvant, diluent or carrier,
   which components (a) and (b) are each provided in a form that is suitable for administration in conjunction with the other.

Examples of therapeutic agents useful in the treatment of autoimmune diseases include the following:
(i) non-steroidal anti-inflammatory drugs (NSAIDs), examples of which include salicylates such as aspirin (acetylsalicylic acid), iflunisal (Dolobid), salicylic acid and its salts and salsalate (Disalcid); propionic acid derivatives such as ibuprofen, dexibuprofen, naproxen, fenoprofen, ketoprofen, dexketoprofen, flurbiprofen, oxaprozin, loxoprofen, pelubiprofen and zaltoprofen; acetic acid derivatives such as indomethacin, tolmetin, sulindac; etodolac; ketorolac; diclofenac; aceclofenac; bromfenac and nabumetone; enolic acid (oxicam) derivatives such as piroxicam; meloxicam; tenoxicam; droxicam; lornoxicam or phenylbutazone; anthranilic acid derivatives (fenamates) such as mefenamic acid, meclofenamic acid, flufenamic acid and tolfenamic acid; or selective COX-2 inhibitors (coxibs) such as celecoxib, parecoxib, lumiracoxib or etoricoxib;
(ii) glucocorticoids, such as prednisone, hydrocortisone and budesonide;
(iii) disease-modifying antirheumatic drugs (DMARDs) such as abatacept, adalimumab, alefacept, anakinra, apremilast, azathioprine, baricitinib, certolizumab pegol, chloroquine, ciclosporin, D-penicillamine, efalizumab, etanercept, filgotinib, golimumab, guselkumab, hydroxychloroquine, infliximab, leflunomide, methotrexate, 6-mercaptopurine, rituximab, sariluman, sulfasalazine, tocilizumab, tofacitinib and ustekinumab;
(iv) cyclosporin;
(v) topical calcineurin inhibitors such as tacrolimus and pimecrolimus;
(vi) anti-CD20 antibodies, such as rituximab, ocrelizumab, obinutuzumab, ofatumumab, ibritumomab tiuxetan, tositumomab, and ublituximab;
(vii) interleukin-17 inhibitors, including secukinumab, ixekizumab and brodalumab,
(vii) IFN receptor agonists, such as emapalumab;
(viii) B-cell activating factor inhibitors, such as belimumab;
(ix) gold compounds, such as sodium aurothiomalate.

Examples of therapeutic agents useful in the treatment of inflammatory diseases include those listed above for the treatment of autoimmune diseases, and immune-selective anti-inflammatory derivatives (ImSAIDS) such as the tripeptide FEG (Phe-Glu-Gly) and its D-isomeric form feG.

Compounds of the invention may have the advantage that they are effective inhibitors of protein or lipid kinases (e.g. a PIM family kinase such as PIM-1, PIM-2 and/or PIM-3). Advantageously, when compounds of the invention are employed in combination with known chemotherapeutic agents (such as those described herein), the components of the combinations may act in a synergistic manner.

Compounds of the invention may also have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties over, compounds known in the prior art, whether for use in the above-stated indications or otherwise.

### Medical Uses and Methods of Treatment

The compounds of the invention are indicated as pharmaceuticals. According to a further aspect of the invention there is provided a compound of the invention, as hereinbefore defined, for use as a pharmaceutical.

Compounds of the invention may inhibit protein or lipid kinases, such as a PIM family kinase such as PIM-1, PIM-2 and/or PIM-3, for example as may be shown in the tests described below and/or in tests known to the skilled person. Thus, the compounds of the invention may be useful in the treatment of those disorders in an individual in which the inhibition of such protein or lipid kinases (e.g. a PIM family kinase such as PIM-1, PIM-2 and/or PIM-3) is desired and/or required.

The term "inhibit" may refer to any measurable reduction and/or prevention of catalytic kinase (e.g. a PIM family kinase such as PIM-1, PIM-2 and/or PIM-3) activity. The reduction and/or prevention of kinase activity may be measured by comparing the kinase activity in a sample containing a compound of the invention and an equivalent sample of kinase (e.g. a PIM family kinase such as PIM-1, PIM-2 and/or PIM-3) in the absence of a compound of the invention, as would be apparent to those skilled in the art. The measurable change may be objective (e.g. measurable by some test or marker, for example in an *in vitro* or *in vivo* assay or test, such as one described hereinafter, or otherwise another suitable assay or test known to those skilled in the art) or subjective (e.g. the subject gives an indication of or feels an effect).

Compounds of the invention may be found to exhibit 50% inhibition of a protein or lipid kinase (e.g. a PIM family kinase such as PIM-1, PIM-2 and/or PIM-3) at a concentration of 100 µM or below (for example at a concentration of below 50 µM, or even below 10 µM, such as below 1 µM), when tested in an assay (or other test), for example as described hereinafter, or otherwise another suitable assay or test known to the skilled person.

Compounds of the invention are thus expected to be useful in the treatment of a disorder in which a protein or lipid kinase (e.g. a PIM family kinase such as PIM-1, PIM-2 and/or PIM-3) is known to play a role and which are characterised by or associated with an overall elevated activity of that protein kinase (due to, for example, increased amount of the kinase or increased catalytic activity of the kinase). Compounds of the invention (alone or in combination with another active) may be shown to be active e.g. in the biochemical assays described herein, may be shown to have predictive activity based on e.g. the phosphorylation assay described herein, and/or may reduce the rate of cell proliferation e.g. as may be shown in the cell proliferation assays described herein (for instance using cancer cell lines (e.g. known commercially available ones), such as those described herein).

Hence, compounds of the invention are expected to be useful in the treatment of a disease/disorder arising from abnormal cell growth, function or behaviour associated with the protein or lipid kinase (e.g. a PIM family kinase such as PIM-1, PIM-2 and/or PIM-3). Such conditions/disorders include cancer, immune disorders, cardiovascular diseases, viral infections, inflammation, metabolism/endocrine function disorders and neurological disorders.

The disorders/conditions that the compounds of the invention may be useful in treating hence include cancer (such as lymphomas, solid tumours or a cancer as described hereinafter), obstructive airways diseases, allergic diseases, inflammatory diseases (such as asthma, allergy, and inflammatory bowel disease such as colitis and Crohn's disease), immunosuppression (such as transplantation rejection), inflammatory and autoimmune diseases, for example rheumatoid arthritis, psoriasis, psoriatic arthritis, osteoarthritis, ankylosing spondylitis, atopic dermatitis, inflammatory bowel diseases such as ulcerative colitis and Crohn's disease, systemic lupus erythematosus, lupus nephritis, multiple sclerosis, peanut allergy, asthma and celiac disease), disorders commonly connected with organ transplantation, AIDS-related diseases and other associated diseases. Other associated diseases that may be mentioned (particularly due to the key role of kinases in the regulation of cellular proliferation) include other cell proliferative disorders and/or non-malignant diseases, such as benign prostate hyperplasia, familial adenomatosis, polyposis, neuro-fibromatosis, bone disorders, atherosclerosis, vascular smooth cell proliferation associated with atherosclerosis, pulmonary fibrosis, arthritis glomerulonephritis and post-surgical stenosis and restenosis. Other disease states that may be mentioned include cardiovascular disease, stroke, diabetes, hepatomegaly, Alzheimer's disease, cystic fibrosis, hormone-related diseases, immunodeficiency disorders, destructive bone disorders, infectious diseases, conditions associated with cell death, thrombin-induced platelet aggregation, chronic myelogenous leukaemia, liver disease, pathologic immune conditions involving T cell activation and CNS disorders, pulmonary arterial hypertension (PAH), chronic obstructive pulmonary disease (COPD), sepsis, systemic sclerosis (scleroderma),

Type 1 diabetes, and hidradenitis suppurativa.

According to a further aspect of the present invention, there is provided a compound of the invention, as hereinbefore defined, for use in the treatment of cancer.

According to a further aspect of the present invention, there is provided use of a compound of the invention, as hereinbefore defined, in the manufacture of a medicament for the treatment of cancer.

According to a further aspect of the present invention, there is provided a compound of the invention, as hereinbefore defined, for use in a method of treatment of cancer, which method comprises administration of a therapeutically effective amount of a compound of the invention, as hereinbefore defined, to a patient suffering from, or susceptible to, such a condition.

According to said aspect of the present invention, there is provided a compound of the invention, as hereinbefore defined, for use in the treatment of cancer.

As stated above, the compounds of the invention may be useful in the treatment of cancer. More, specifically, the compounds of the invention may therefore be useful in the treatment of a variety of cancer including, but not limited to: carcinoma such as cancer of the bladder, breast, colon, kidney, liver, lung (including non-small cell cancer and small cell lung cancer), esophagus, gall-bladder, ovary, pancreas, stomach, cervix, thyroid, prostate, skin, squamous cell carcinoma, testis, genitourinary tract, larynx, glioblastoma, neuroblastoma, keratoacanthoma, epidermoid carcinoma, large cell carcinoma, non-small cell lung carcinoma, small cell lung carcinoma, lung adenocarcinoma, bone, adenoma, adenocarcinoma, follicular carcinoma, undifferentiated carcinoma, papilliary carcinoma, seminoma, melanoma, sarcoma, bladder carcinoma, liver carcinoma and biliary passages, kidney carcinoma, myeloid disorders, lymphoid disorders, hairy cells, buccal cavity and pharynx (oral), lip, tongue, mouth, pharynx, small intestine, colon-rectum, large intestine, rectum, brain and central nervous system, Hodgkin's and leukaemia; hematopoietic tumors of lymphoid lineage, including leukaemia, acute lymphocitic leukaemia, acute lymphoblastic leukaemia, B-cell lymphoma, T-cell-lymphoma, mantle cell lymphoma, Hodgkin's lymphoma, non-Hodgkin's lymphoma, hairy cell lymphoma and Burkett's lymphoma; hematopoietic tumors of myeloid lineage, including acute and chronic myelogenous leukaemias, myelodysplastic syndrome and promyelocytic leukemia; tumors of mesenchymal origin, including fibrosarcoma and rhabdomyosarcoma; tumours of the central and peripheral nervous system, including astrocytoma, neuroblastoma, glioma and schwannomas; and other tumours, including melanoma, seminoma, teratocarcinoma, osteosarcoma, xeroderma pigmentosum, keratoxanthoma, thyroid follicular cancer and Kaposi's sarcoma.

According to a further aspect of the present invention, there is provided use of a compound of the invention, as hereinbefore defined, in the manufacture of a medicament for the treatment of an autoimmune disease or an inflammatory disease.

According to a further aspect of the present invention, there is provided a compound of the invention, as hereinbefore defined, for use in a method of treatment of an autoimmune disease or an inflammatory disease, which method comprises administration of a therapeutically effective amount of a compound of the invention, as hereinbefore defined, to a patient suffering from, or susceptible to, such a condition.

According to said aspect of the present invention, there is provided a compound of the invention, as hereinbefore defined, for use in the treatment of an autoimmune disease or an inflammatory disease.

Examples of autoimmune disease and/or inflammatory diseases include but are not limited to coeliac disease, psoriasis, inflammatory bowel diseases such as colitis and Crohn's disease, multiple sclerosis, rheumatoid arthritis, systemic lupus erythematosus, aplastic anaemia, myocarditis, autoimmune hepatitis, primary biliary cholangitis, alopecia areata, autoimmune urticaria, perphigus vulgaris, autoimmune polyendocrine syndrome (APS), autoimmune pancreatitis, type 1 diabetes, autoimmune thyroiditis, Sjögren's syndrome, autoimmune haemolytic anaemia, gastritis vasculitis, granlumatosis with polyangitis, autoimmune uveitis, myasthenia gravis, acute disseminated encephalomyelitis, relapsing polychrondritis, Lambert-Eaton myasthenic syndrome, Hashimoto's encephalopathy, Felty syndrome, autoimmune thyroiditis, Graves' disease, nephritis, bullous pemphigoid, dermatitis, epidermolysis bullosa acquisita, linear IgA disease, autoimmune lymphoprolifeative syndrome, autoimmune neutropenia, autoimmune thrombocytopenis purpura, cold agglutinin disease, Evans syndrome, pernicious anaemia, Still's disease, psoriatic arthritis, rheumatic fever, Guillain-Barré syndrome, Ord's thyroiditis, ankylosing spondylitis, atopic dermatitis, systemic sclerosis (scleroderma), hidradenitis suppurativa, lupus nephritis, peanut allergy, and asthma.

Further, the protein or lipid kinases (e.g. a PIM family kinase such as PIM-1, PIM-2 and/or PIM-3) may also be implicated in the multiplication of viruses and parasites. They may also play a major role in the pathogenesis and development of neurodegenerative disorders. Hence, compounds of the invention may also be useful in the treatment of viral conditions, parasitic conditions, as well as neurodegenerative disorders.

Compounds of the invention are indicated both in the therapeutic and/or prophylactic treatment of the above-mentioned conditions.

According to a further aspect of the present invention, there is provided a compound of the invention, as hereinbefore defined, for use in a method of treatment of a disease (e.g. cancer or another disease as mentioned herein) which is associated with the inhibition of protein or lipid kinase (e.g. a PIM family kinase such as PIM-1, PIM-2 and/or PIM-3) is desired and/or required (for example, a method of treatment of a disease/disorder arising from abnormal cell growth, function or behaviour associated with protein or lipid kinases, e.g. a PIM family kinase such as PIM-1, PIM-2 and/or PIM-3), which method comprises administration of a therapeutically effective amount of a compound of the invention, as hereinbefore defined, to a patient suffering from, or susceptible to, such a condition.

"Patients" include mammalian (including human) patients. Hence, the method of treatment discussed above may include the treatment of a human or animal body.

The term "effective amount" refers to an amount of a compound, which confers a therapeutic effect on the treated patient. The effect may be objective (e.g. measurable by some test or marker) or subjective (e.g. the subject gives an indication of or feels an effect).

### Abbreviations

In the sections which follow, the following abbreviations are used:
- 1,2-DCE: 1,2-dichloroethane
- ATP: adenosine triphosphate
- DCM: dichloromethane
- EtOAc: ethyl acetate
- Et₃N: triethylamine
- h: hours
- min: minutes
- HPLC: high performance liquid chromatography
- MeOH: methanol
- mw: microwave
- nBuOH: n-butanol
- NMR: nuclear magnetic resonance
- AcCN: acetonitrile
- rt: room temperature
- c-Hex: cyclohexane
- DMSO: dimethylsulfoxide
- EtOH: ethanol
- AcOH: acetic acid
- Rt: retention time
- LCMS: liquid-chromatography-mass spectrometry
- ESI: electrospray ionization
- p-TsOH: p-toluenesulfonic acid
- Et₂O: diethyl ether
- THF: tetrahydrofuran
- Pd₂dba₃: tris(dibenzylideneacetone)-dipalladium(0)
- Xantphos: (9,9-dimethyl-9H-xanthene-4,5-diyl)bis(diphenylphosphane)
- HEPES: 4-(2-hydroxyethyl)-1-piperazineethanesulfonic acid
- EGTA: ethylene glycol-bis(β-aminoethyl ether)-*N*,*N*,*N*',*N*'-tetraacetic acid
- DMEM: Dulbecco's modified Eagle's medium

The following Examples illustrate the invention. The preparation of the intermediates is described in the Intermediates section below.

### Intermediates

Intermediate 1: 5-Chloro-3-(4-fluoro-3-trifluoromethyl-phenyl)-6-methyl-7,8-dihydro-6H-9-oxa-1,2,3a,4,6-pentaaza-cyclopenta[a]naphthalene

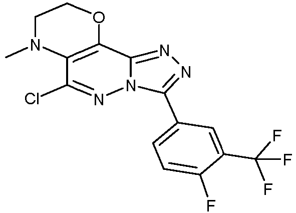

5,8-Dichloro-4-methyl-3,4-dihydro-2H-pyridazino[4,5-b][1,4]oxazine (Intermediate 12 as disclosed in WO2011/080510) (9 g, 41 mmol) and 4-fluoro-3-trifluoromethylbenzoic acid hydrazide (Intermediate 7) (13.6 g, 61 mmol) were heated in nBuOH (180 ml) at 170 °C for 20 h. The red-dark mixture was concentrated *in vacuo,* and the residue was purified by automated flash chromatography (SiO₂, eluents c-Hex/EtOAc from 75:25 to 0:100 to afford the desired regioisomer, Intermediate 1 (2.51 g; 16% yield). LCMS1 (ESI): Rt = 5.7 min, m/z = 388.20 [M+ H]⁺. ¹H NMR (300 MHz, DMSO-*d₆*) δ 8.72 (m, 2H), 7.89 (t, J = 9.6 Hz, 1H), 4.60 (t, J = 4.0 Hz, 2H), 3.34 (m, 3H), 2.91 (s, 3H).

Intermediate 2: 4-Allyloxy-3,6-dichloropyridazine

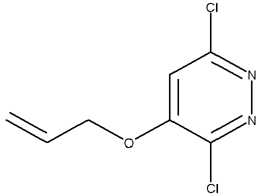

3,4,6-trichloropyridazine (7.5g, 40.889 mmol) and Cs₂CO₃ (1.6 equiv, 69.51 mmol, 22.65g) were mixed in acetonitrile (18 2 mL) and then heated at 65 °C. Allyl alcohol (1.5 equiv, 61.33mmol, 4.171 mL) was added dropwise. The mixture was heated at 65°C for 18 h and then the solvent was concentrated under vacuum. The residue was taken into DCM-water. Layers were separated and the organic phase was washed with brine, dried (Na₂SO₄) and concentrated under vacuum. The crude was purified by flash chromatography (SiO₂, eluents 0-50% EtOAc in cyclohexane) to afford 4-allyloxy-3,6-dichloropyridazine, intermediate 2 (3.451 g, 41% yield) as a white solid. ¹H NMR (300 MHz, Chloroform-d) δ 6.92 (s, 1H), 6.03 (ddt, J = 17.3, 10.5, 5.3 Hz, 1H), 5.59 - 5.37 (m, 2H), 4.72 (dt, J = 5.3, 1.5 Hz, 2H).

Intermediate 3: 8-Allyloxy-6-chloro-3-(3-trifluoromethoxy-phenyl)-[1,2,4]triazolo[4,3-b]pyridazine

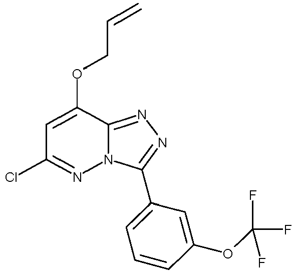

Intermediate 2 (3.451 g, 16.8 mmol) and 3-trifluoromethoxy-benzoic acid hydrazide (CAS 321195-88-4, available as PC061 from Apollo Scientific) (4.076 g, 18.51 mmol, 1.1mmol) were dissolved in dioxane (50 mL) and then p-TsOH (3.201 g, 16.83 mmol, 1.0 eq) was added. The mixture was refluxed for 4 h. Dioxane was removed under vacuum and the residue was taken into DCM and aqueous saturated solution of NaHCO₃. The layers were separated and the organic layer was washed with brine, then dried with sodium sulfate and concentrated under vacuum. The crude was purified by flash chromatography (SiO₂, eluents c-Hex-EtOAc from 0-50% of EtOAc) to afford Intermediate 3 (0.576 g, 9% yield) as a white solid. ¹H NMR (300 MHz, CDCl₃) δ 8.44 (dt, J = 7.9, 1.3 Hz, 1H), 8.39 (m 1H), 7.59 (t, J = 8.1 Hz, 1H), 7.42 - 7.34 (m, 1H), 6.48 (s, 1H), 6.13 (ddt, J = 17.2, 10.9, 5.6 Hz, 1H), 5.64 - 5.43 (m, 2H), 5.02 (dt, J = 5.6 Hz, 0.9 Hz, 2H).

Intermediate 4: 7-Allyl-6-chloro-3-(3-trifluoromethoxy-phenyl)-[1,2,4]triazolo[4,3-b]pyridazin-8-ol

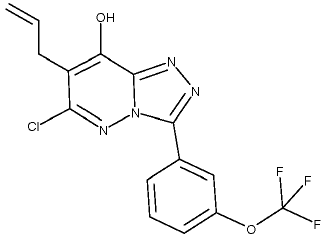

Intermediate 3 (576 mg, 1.54 mmol) in 1,2-DCE (10 mL) in a microwave vial was heated at 165 °C for 1 h under microwave irradiation. Precipitation was observed on the reaction mixture. The solvent was removed under vacuum and the solid was filtered and washed with Et₂O to afford Intermediate 4 (0.329 g, 57% yield) as a white solid. ¹H NMR (300 MHz, CDCl₃) δ: 8.79 (dt, J = 7.9, 1.2 Hz, 1H), 8.41 (m, 1H), 7.64 (t, J = 8.1 Hz, 1H), 7.35 (m, 1H), 5.8 (m, 2H), 5.2-5.07 (m, 2H), 3.69 (m, 2H).

Intermediate 5: 6-Chloro-7-(3-hydroxy-propyl)-3-(3-trifluoromethoxy-phenyl)-[1,2,4]triazolo [4,3-b]pyridazin-8-ol

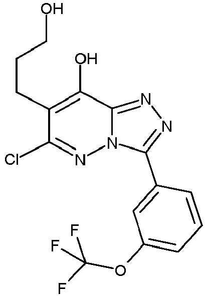

Borane-dimethylsulfide complex (2M in THF, 1.84 mL) was added at 0 °C to a suspension of Intermediate 4 (0.329 g, 0.887 mmol) in dry THF (20 mL), and the mixture was stirred overnight. The reaction was quenched carefully at 0 °C with water, and sodium perborate (683 mg) was added. The stirring was continued at rt for 2.5 h. The mixture was acidified with 1M aq HCl and then extracted with EtOAc. The organic layer was dried (Na₂SO₄) and concentrated to give a mixture of products: Intermediate 5, and its isomer 6-Chloro-7-(2-hydroxy-propyl)-3-(3-trifluoromethoxyphenyl)-[1,2,4] triazolo[4,3-b]pyridazin-8-ol as a white solid (0.420 g), which were used as such in next reaction step. LCMS (ESI): Rt = 4.98 and 5.1 min, m/z =389.02 [M+ H]⁺.

Intermediate 6: 5-Chloro-3-(3-trifluoromethoxy-phenyl)-7,8-dihydro-6H-9-oxa-1,2,3a,4-tetraaza-cyclopenta[a]naphthalene

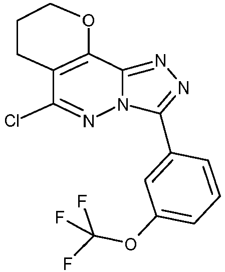

Vilsmeier's reagent (0.207 g, 1.62 mmol, 1.5 eq) was added at rt to a suspension of a mixture of isomers which contains intermediate 5 (0.420 g; 1.08 mmol, 1.0 eq) in DCM (10 mL). After 10 min a solution is obtained and Et₃N (0.753 mL) was added and the mixture was stirred overnight. Solvent was removed under vacuum and the crude was purified by column chromatography to separate both isomers (SiO₂, eluents from 0-4% MeOH in DCM) and to afford Intermediate 6 (0.243 g, 60% yield) as a yellow solid. ¹H NMR (300 MHz, CDCl₃) δ 8.42 (m, 1H), 8.38 (s, 1H), 7.57 (dd, t, J = 8.1 Hz, 1H), 7.35 (m, 1H), 4.55 (m, 2H), 2.80 (t, J = 6.4 Hz, 2H), 2.24 (m, 2H).

Intermediate 7: 4-Fluoro-3-trifluoromethyl-benzoic acid hydrazide

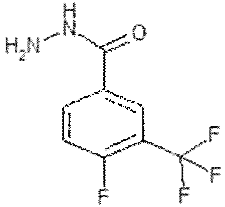

A mixture of 4-fluoro-3-(trifluoromethyl)benzoic acid (15 g, 72 mmol) and SOCl₂ (10.5 mL, 144 mmol) was heated at reflux for 2 h (80°C). The reaction mixture was concentrated under vacuum; the residue was dissolved in toluene and evaporated under reduced pressure to give a crude of 4-fluoro-3-trifluoromethyl-benzoyl chloride which was dried under vacuum overnight and then used without further purification. To a solution of N₂H₄ H₂O (9.6 mL) in anhydrous DCM (45 mL) at 0 °C was added dropwise 4-fluoro-3-trifluoromethyl-benzoylchloride dissolved in DCM (45 mL) and temperature of reaction was allowed to rt over 2 h. The solvent was removed and hot water was added to the white residue to give a precipitate which was filtered and washed with more hot water. A white solid was dried in the fume hood overnight to provide a white solid. Water was extracted with DCM, organic phases were dried over MgSO₄, filtered and dryness to give 4-Fluoro-3-trifluoromethyl-benzoic acid hydrazide compound (1.34 g). Over yield (two steps) 61%. ¹H NMR (300 MHz, DMSO) δ 10.05 (s, 1H), 8.29 - 7.96 (m, 2H), 7.63 (t, J = 9.7 Hz, 1H), 4.62 (s, 2H).

### Examples

### Example 1: 1-Methyl-4-[6-methyl-3-(3-trifluoromethoxy-phenyl)-7,8-dihydro-6H-9-oxa-1,2,3a,4,6-pentaaza-cyclopenta[a]naphthalen-5-ylamino]-cyclohexanol

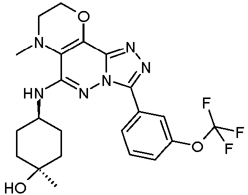

A deoxygenated solution of 5-Chloro-6-methyl-3-(3-trifluoromethoxy-phenyl)-7,8-dihydro-6H-9-oxa-1,2,3a,4,6-pentaaza-cyclopenta[a]naphthalene (disclosed as Intermediate 18 in WO2011/080510) (0.5 g; 1.296 mmol), *cis-*4-amino-1-methyl-cyclohexanol (0.251 g; 1.94 mmol), NaOtBu (0.224 g; 2.33 mmol), XantPhos (0.075 g; 0.13 mmol) and Pd₂dba₃ (0.059g; 0.065 mmol) in dioxane (2.0 mL) was heated at 100 °C for 1 h under microwave conditions. The reaction was performed in nine batches of 500 mg of the intermediate. The different batches were combined, and solvent was removed under vacuum. The crude was purified by column chromatography (SiO₂, eluent from 0-8% MeOH-NH₃ in DCM), and then triturated from EtOAc to afford the compound of Example 1 (1.880 g, 34% yield) as a white solid. LCMS1 (ESI): Rt = 5.49 min, m/z =479.02 [M+ H]⁺. ¹H NMR (300 MHz, CDCl₃) δ 8.61 (s, 1H), 8.47 (d, J = 8.0 Hz, 1H), 7.52 (t, J = 8.1 Hz, 1H), 7.29 (m, 1H), 4.96 (d, J = 7.1 Hz, 1H), 4.47 (m, 2H), 3.80 (m, 1H), 3.20 (m, 2H), 2.73 (s, 3H), 2.07 (m, 2H), 1.71 (m, 6H), 1.31 (s, 3H).

### Example 2: 4-[3-(4-Fluoro-3-trifluoromethyl-phenyl)-6-methyl-7,8-dihydro-6H-9-oxa-1,2,3a,4,6-pentaaza-cyclopenta[a]naphthalen-5-ylamino]-1-methyl-cyclohexanol

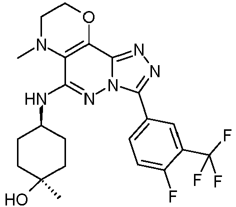

Intermediate 1 (1 g, 2.58 mmol) was dissolved in n-BuOH (12.5 mL), Et₃N (1.1 mL; 7.74 mmol) and *cis*-4-amino-1-methyl-cyclohexanol (1 g; 7.74 mmol) were added. The mixture was heated at 180 °C in a pressure tube. After overnight stirring, the reaction finished and the solvent was evaporated to get a residue which was purified by automated flash chromatography (SiO₂, eluents DCM/MeOH from 100 to 96:4) to afford the compound of Example 2 as a white solid (444 mg; 36% yield). LCMS1 (ESI): Rt = 5.5 min, m/z =481.02 [M+ H]⁺. ¹H NMR (300 MHz, DMSO) δ 8.97 (d, J = 5.7 Hz, 1H), 8.67 (m, 1H), 7.71 (t, J = 12 Hz, 1H), 6.25 (d, J = 7.5 Hz, 1H), 4.43 (t, J = 3.9 Hz, 2H), 4.09 (s, 1H), 3.68 (m, 1H), 3.20 (m, 2H), 2.66 (s, 3H), 1.81 (m, 4H), 1.61 (m, 2H), 1.36 (m, 2H), 1.14 (s, 3H).

### Example 3: 1-Methyl-4-[3-(3-trifluoromethoxy-phenyl)-7,8-dihydro-6H-9-oxa-1,2,3a,4-tetraaza-cyclopenta[a]naphthalen-5-ylamino]-cyclohexanol

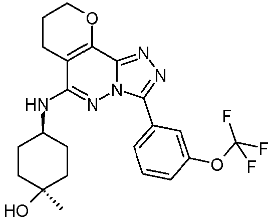

A solution of Intermediate 6 (0.243 g; 0.655 mmol) and cis-4-amino-1-methyl-cyclohexanol (0.254 g; 1.96 mmol, 3.0 eq) and Et₃N (183µl, 2.0 eq) in n-BuOH (8.0 mL) was heated at 185 °C in an oil bath. Solvent was removed under vacuum and the crude was purified by column chromatography (SiO₂, from 0-6% MeOH-NH₃ in DCM) to afford the desired product of Example 3 (50 mg, 16% yield). LCMS1 (ESI): Rt = 5.547 min, m/z = 464.20 [M+ H]⁺. ¹H NMR (300 MHz, DMSO-*d₆*) δ 8.67 (s, 1H), 8.42 (d, J = 8.1 Hz, 1H), 7.70 (t, J = 8.1 Hz, 1H), 7.50 (d, J = 8.2 Hz, 1H), 6.40 (d, J = 7.4 Hz, 1H), 4.40 (m, 2H), 4.12 (s, 1H), 3.70 (m, 1H), 2.08 (m, 2H), 1.79 (t, J = 7.4 Hz, 4H), 1.65 (m, 2H), 1.40 (m, 2H), 1.14 (s, 3H).

### Biological Examples

### Biological Example 1 - Protein PIM kinase assays

PIM kinase activities were measured by using the commercial ADP Hunter^{™} Plus assay (DiscoveRx Ref. #33-016), a homogeneous assay measuring ADP accumulation, as a universal product of kinase activity. PIM-1 and PIM-2 proteins were obtained by purification as it is described by Martinez-Gonzalez et. al. Eur. J. Med Chem. 2019, 168, 87-109 and PIM-3 was acquired from Millipore (Cat#14-738). The assay was done following general manufacturer recommendations and adapting protein and substrates concentrations to optimal conditions. Kinase buffer was 15 mM HEPES, pH 7.4, 20 mM NaCl, 1 mM EGTA, 0.02% Tween-20, 10 mM MgCl₂ and 0.1 mg/mL LBGG (bovine γ-globulin). All PIM kinases assays were done at 100 µM PIM tide (ARKRRRHPSGPPTA), as peptide substrate, and 100 µM ATP. Protein concentration was 50, 350 and 200 pg/µl for PIM-1, 2 and 3, respectively. In order to calculate the IC₅₀ of described compounds, serial 1:3 dilutions were prepared and the reaction started by addition of ATP. Incubation was done for 1 h at 25 °C. Reagents A and B (DiscoveRx) were sequentially added to the wells and plates were incubated for 30 min at 37 °C. Fluorescence counts were read in a Victor instrument (Perkin Elmer) with the recommended settings (544 and 580 nm as excitation and emission wavelengths, respectively). Values were plot against inhibitor concentration and fit to a sigmoid dose-response curve using Activity Base software from IDBS.

The results are shown in Table 1. Comparative Example 1 is Example 19 of WO2011/080510, which is believed to represent the structurally closest prior art compound to those of the present invention. In Table 1, **** represents an IC₅₀ below 1nM, *** represents an IC₅₀ between 1 and 5 nM, ** represents an IC₅₀ between 5 and 10 nM, and ** represents an IC₅₀ between 10 and 50 nM,

**Table 1**

| **Compound** | **PIM-1** | **PIM-2** | **PIM-3** |
|---|---|---|---|
| Example 1 | *** | ** | ** |
| Example 2 | **** | *** | *** |
| Example 3 | **** | ** | *** |
| Comparative Example 1 | *** | ** | * |

The results show that the activity of the compounds of the invention against PIM-1, PIM-2 and PIM-3 kinases is at least comparable to those of the closest prior art compound. The compounds can therefore be expected to be useful in the treatment of diseases mediated by these PIM kinases, as outlined herein.

### Biological Example 2 - BAD S112 Phosphorylation inhibition assay

The efficacy of the compounds of the invention on the inhibition of BAD phosphorylation was measured by an in Cell ELISA.

Materials: H1299 cells overexpressing PIM-1 (H1299Pim1). DMSO Plates: 96-well-Polystyrene, Untreated, Round-Bottom plates from Costar (Cat #3797). Cell Plates: 96-Flat bottom biocoated with Poly-D-Lysin plates with lid from Becton Dickinson (Cat#354651). Cell Culture Medium: DMEM high glucose, 10% Fetal Bovine Serum, 2mM L-Glutamine, P/S. Antibodies: phospho BAD S112 antibody from Cell Signaling (cat. #9291S), anti-rabbit conjugated with peroxidase from Amersham (cat.#3619). Reagent: SuperSignal ELISA femto from Pierce (cat.#1001110).

Procedure: Cells were seeded in 15000 cells per 200 µl per well into 96-well plates and incubated for 16 h at 37 °C, 5% CO₂. On day two, nine serial 1:2 compound dilutions were made in DMSO in a 96-well plate. The compounds were added to duplicate wells in 96-well cell plates using a FX BECKMAN robot (Beckman Coulter) and were incubated at 37 °C under CO₂ atmosphere in medium without fetal bovine serum (FBS). After 4 hours, relative levels of BAD S112 phosphorylation were measured in Cell ELISA using SuperSignal ELISA Femto substrate (Pierce) and read on VICTOR (Perkin Elmer). EC₅₀ values were calculated using ActivityBase from IDBS.

The results are shown in Table 2. Comparative Example 1 is Example 19 of WO2011/080510.

**Table 2**

| **Compound** | **BAD PH 1299PIM 1 ELIWO (M)** |
|---|---|
| Example 1 | 8.30E-09 |
| Example 2 | 7.11E-09 |
| Example 3 | 4.15E-09 |
| Comparative Example 1 | >1E-05 |

From the above, it can be concluded that the compounds of the Examples improve the cellular modulation of a PIM biomarker, therefore conferring the potential for the compounds to improve the response of the cells to the treatment with the PIM inhibitor.

### Biological Example 3 - Antiproliferative Assay

The activity of the compounds of the invention against proliferation of the cancer cell lines A549 (used as a model for the study of lung cancer) and MiaPaca (used as a model for the study of pancreatic cancer) were tested and compared with those of the closest prior art compound (Example 19 of WO2011/080510).

The cells were harvested just before reaching confluency, counted with a haemocytometer and diluted with media. Cells were then seeded in 96-well microtiter plates at a density of 4,000 cells/well. Cells were incubated for 24 hours before adding the compounds.

Compounds were weighed out and diluted with DMSO to a final concentration of 10 mM. From here a "mother plate" with serial dilutions was prepared at 200X the final concentration in the culture. The final concentration of DMSO in the tissue culture media should not exceed 0.5%. The appropriate volume of the compound solution (2 µl) was added automatically (Beckman FX 96 tip) to 0.2 ml media to make it up to the final concentration for each drug.

Each concentration was assayed in duplicate. Cells were exposed to the compounds for 72 h and then processed for CellTiter-Glo^{®} Luminescent Cell Viability Assay (Promega) read out according to manufacturer's Instruction and read on EnVision (Perkin Elmer). GI₅₀ values were calculated using ActivityBase from IDBS.

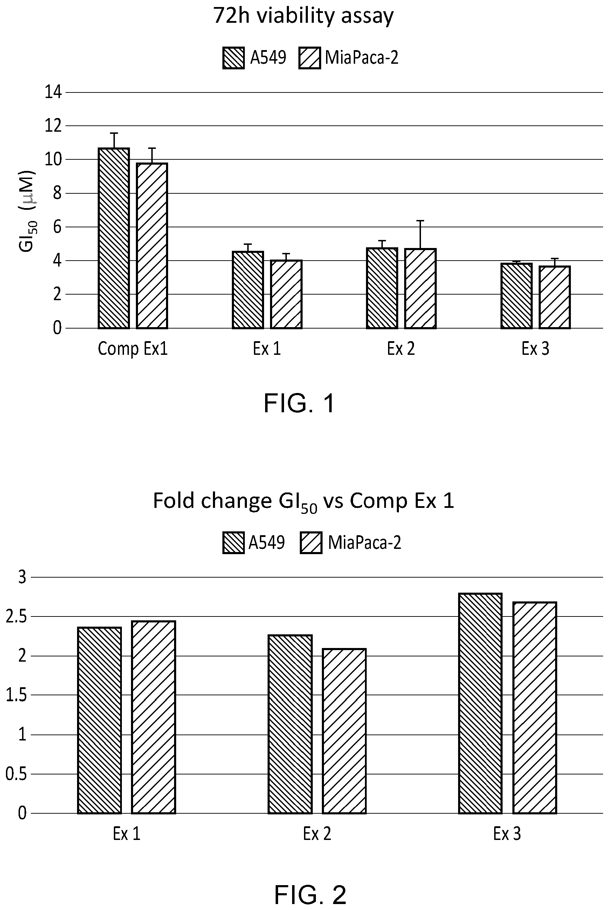

The results are shown in Figures 1 and 2. Figure 1 shows the viability of the cells in each cell line after 72 hours in respect of the three Example compounds and Comparative Example 1 (Example 19 of WO2011/080510). Figure 2 compares the fold change of GI₅₀ for the three Example compounds of the invention against these cell lines with Comparative Example 1.

The results show that all three Example compounds of the present invention exhibit surprisingly improved anti proliferative activity against both cell lines, compared with the structurally closest compound of Comparative Example 1. The compounds therefore have the potential for development as more potent pharmaceuticals for the treatment of cancers, in particular lung and pancreatic cancer, at increased *in vivo* efficacy in patients compared with the closest compound of Comparative Example 1.

### Biological Example 4 - Pharmacokinetics

Female BALB/c mice, 10 weeks old were used (n=3 per time point) for the *in vivo* clearance determination. The compounds were formulated for intravenous injections (i.v.) in 10% N-methylpyrrolidone (NMP) /50% polyethylene glycol PEG300/ 40% NaCl 0.9% and for oral injections (PO) 10% NMP/90% PEG300. Plasma samples were collected following a single i.v. administration at 0.08, 0.25, 0.5, 1 and 4 hours, and after a single PO administration at 0.08, 0.16, 0.25, 0.5, 1, 4, 8 and 24 hours.

The extraction of compound from plasma was achieved by solid phase extraction followed by high performance liquid chromatography/mass spectrometry (Agilent 1100, Applied Biosystems API2000) analysis. The amount of inhibitor and the internal standard in each mouse plasma sample were quantified based on calibration curves generated using standards of known concentrations of compound.

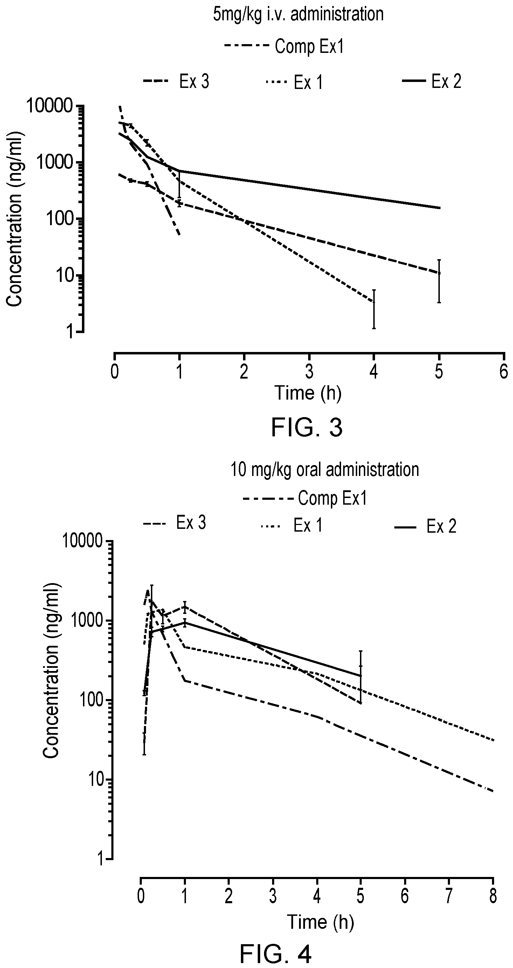

The results are shown in Figures 3 and 4. Figure 3 shows the concentration of each compound as a function of time when the compounds are administered at 5 mg/kg intravenously, and Figure 4 shows the concentration of each compound as a function of time when the compounds are administered at 10 mg/kg orally.

The results show that all three Example compounds of the present invention exhibit surprisingly improved pharmacokinetic activity, compared with the structurally closest compounds of Comparative Example 1. This means the compounds would therefore have greater exposure to the site of action *in vivo,* conferring the potential for development as pharmaceuticals having improved efficacy *in vivo* or comparable efficacy at lower doses *in vivo,* compared with Comparative Example 1.

### Biological Example 5 - Combination Antiproliferation Data

The *in vitro* anti-proliferation activity of combinations of the compound of Example 1 in combination with a number of known anti-cancer compounds was measured using the same methods generally described above for Biological Example 3. A combination index score was calculated by the Chou and Talalay method (CalcuSyn software, Biosoft).

The combination partner compounds were as follows:
the PI3K inhibitor pictisilib (GDC-0941);
the MEK inhibitor merdametinib (PD-0325901); and
the EGFR and ErbB2 inhibitor lapatinib

The results are shown in Table 3. In this Table, ++++ indicates a very strong synergistic effect; +++ a strong synergistic effect; and ++ a synergistic effect.

**Table 3**

| **Combination Agent** | **Cell Lines** | **Disease** | **C.I.** | **Synergy** |
|---|---|---|---|---|
| Lapatinib | T47D | Breast Cancer | 0.27 | +++ |
| Lapatinib | A549 | Lung Cancer | 0.47 | ++ |
| Lapatinib | HCT116 | Colon Cancer | 0.51 | ++ |
| Lapatinib | DU145 | Prostate Cancer | 0.52 | ++ |
| Lapatinib | Jeko-1 | Mantle Cell Lymphoma | 0.70 | ++ |
| Merdametinib | SKMEL19 | Melanoma | 0.038 | ++++ |
| Merdametinib | DU145 | Prostate Cancer | 0.55 | ++ |
| Merdametinib | HT29 | Colon Cancer | 0.62 | ++ |
| Pictisilib | DU145 | Prostate Cancer | 0.24 | +++ |
| Pictisilib | U87MG | Glioblastoma | 0.46 | ++ |
| Pictisilib | SKMEL19 | Melanoma | 0.54 | ++ |

The results show that the combination of the compound of Example 1 with these known anti-cancer compounds results in synergistic antiproliferative activity.

### Biological Example 6 - Further Anti-proliferative Assay

The activity of the compounds of the invention against proliferation of the cancer cell lines MV4:11 (used as a model for the study of acute myeloid leukaemia), HT-29 (used as a model for the study of colon cancer), Jeko1 (used as a model for the study of mantle cell lymphoma) and SKMEL19 (used as a model for the study of melanoma) were tested and compared with those of the closest prior art compound Comparative Example 1 (Example 19 of WO2011/080510). The materials and methods used are the same as in Biological Example 3.

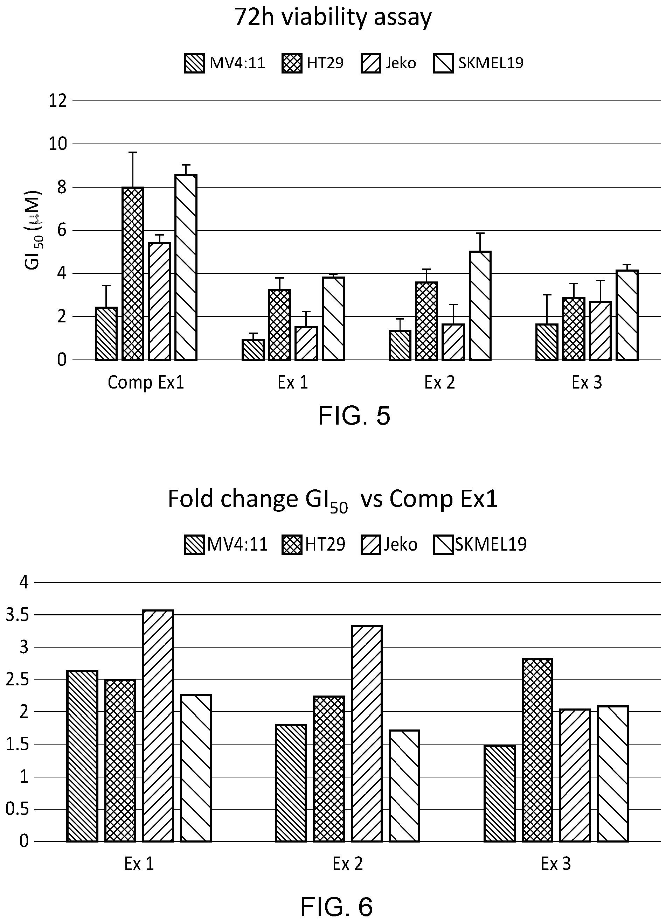

The results are shown in Figures 5 and 6. In particular, Figure 5 illustrates the anti-proliferative activity of the three Example compounds of the invention, showing the viability of the cells in the MV4:11 (acute myeloid leukaemia), HT-29 (colon cancer), Jeko1 (mantle cell lymphoma) and SKMEL19 (melanoma) cell lines 72 hours after treatment with the three Example compounds and with Comparative Example 1, and Figure 6 compares the fold change of GI50 against these cell lines when treated with the three compounds of the invention and with Comparative Example 1.

The results show that all three Example compounds of the present invention exhibit surprisingly improved anti-proliferative activity against the four cell lines, compared with the structurally closest compound of Comparative Example 1. The compounds therefore have the potential for development as more potent pharmaceuticals for the treatment of cancers, in particular acute myeloid leukaemia, colon cancer, mantle cell lymphoma and melanoma, at increased in vivo efficacy in patients compared with the closest compound of Comparative Example 1.

### Biological Example 7 - in vitro potency against breast cancer cell lines

The potency of the compound of Example 1 against a range of cell lines for breast cancer was evaluated. Staurosporine (STS) (from Sigma, Cat. No. S4400-1MG, Lot. No. #115M4047V) and dactolisib (BEZ-235) were used as references.

### Materials

RPMI1640 (from Invitrogen, Cat. No. 11875-093; Lot. No.1811359); fetal bovine serum (FBS) (from Invitrogen, Cat. No. 10099-141, Lot. No. 1652792); Hybricare (from ATCC; Cat.No.46-X; Lot No. 61939036); Insulin, Human Recombinant Zinc (Cat.No.12585-014, Lot.No.1758396); L-glutamine (from Invitrogen, Cat. No. 25030-081, Lot. No. 1532081); 0.25% Trypsin-ethylenediaminetetraacetic acid (EDTA) (from Invitrogen, Cat. No. 25200-072, Lot. No. 1806021); Penicillin-Streptomycin solution (from Hyclone, Cat. No. SV30010, Lot. No. J160016); Glutamax (from Gibco, Cat. No. 35050-061; Lot. No. 1715705); DMSO (from Sigma, Cat. No. 276855-1L. Lot. No. #STBD8882V); 96-well plate, white wall with clear bottom, tissue culture treated (from Corning, Cat. No. CLS3903; Lot.No.16816002); 96 well, Square V-Bottom (from Costar, Cat.No.3960; Lot.No.04914002); CellTiter Glo assay kit (from Promega, Cat. No. G7571, Lot. No. 0000126342). Staurosporine (from Sigma, Cat. No. S4400-1MG, Lot. No. #115M4047V)

### Cell lines - as shown in Table 4 below.

**Table 4**

| **Assay condition: Cell line** | **Vendor** | **Cat#** | **Description** | **Growth properties** | **Complete medium** | **Incubation time (h)** | **Seeding Density** |
|---|---|---|---|---|---|---|---|
| HCC1954 | ATCC | CRL-2338 | breast, ductal carcinoma | adherent | RPMI-1640 +10%FBS | 72 | 3000 |
| HCC38 | ATCC | CRL-2314 | breast, primary ductal carcinoma | adherent | RPMI-1640 +10%FBS | 72 | 1600 |
| HCC1143 | ATCC | CRL-2321 | breast, primary ductal carcinoma | adherent | RPMI-1640 +10%FBS | 72 | 3500 |
| MDA-MB-361 | ATCC | HTB-27 | breast, adenocarcinoma | adherent | RPMI-1640 +10%FBS | 72 | 4000 |
| T-47D | ATCC | HTB-133 | breast, ductal carcinoma | adherent | RPMI-1640 +0.2 Units/ml insulin+10%FBS | 72 | 4000 |
| BT-474 | ATCC | HTB-20 | breast, ductal carcinoma | adherent | Hybricare+10%FBS | 72 | 12000 |

### Experimental Methods

### Celltiter Glo Assay

### Cell Seeding:

To prepare the complete medium FBS and appropriate additives were added according to the information sheet provided by the vendor and mixed gently. The cell name and complete medium and passage number marked on the flask were checked, and the culture medium removed and discarded using a vacuum pump.

The cell layer was briefly rinsed with 0.25% (w/v) Trypsin-0.038% (w/v) EDTA solution to remove all traces of serum that contains trypsin inhibitor. 3.0 ml of Trypsin-EDTA solution was added to the flask and the cells observed under an inverted microscope until cell layer is dispersed. 8.0 ml of complete growth medium was added and the cells aspirated by gently pipetting. The cell suspension was transferred to a centrifuge tube and centrifuged at 800-1000 rpm for 3-5 minutes. The supernatant was discarded using a vacuum pump, and an appropriate volume of complete medium added. The cell pellet was suspended by gently pipetting, and the cell numbers counted with Vi-cell XR and the cells adjusted to appropriate density. 100 µL of cell suspension to 96-well opaque-walled clear bottom plates was added according to the planned plate layout and the plates placed in the CO₂ incubator overnight.

### Compound preparation - shown in Table 5 below.

**Table 5**

| | Stock Conc [mM] | Source | Dilution scheme | Dilution Solvent | Start Working Conc [mM] | Instruction | Final Start Conc [µM] |
|---|---|---|---|---|---|---|---|
| STS | 2 | Sigma | 5xdilution | DMSO | 0.4 | 3xserial dilution, 10pt | 2 |
| Dactolisib | 10 | Inflection | 5xdilution | DMSO | 2 | 3xserial dilution, 10pt | 10 |
| Example 1 | 10 | Inflection | 5xdilution | DMSO | 2 | 3xserial dilution, 10pt | 10 |

### Compound Plate preparation and addition:

a. Plate preparation of testing articles: 10 mM stock solution was prepared in DMSO. The start working solution and 10-point with 3-fold serial dilutions were prepared with DMSO according to Table 5.
b. Staurosporine plate preparation: 0.4 mM Staurosporine was prepared in DMSO at working concentration.
c. Compound addition: 0.5µL of the DMSO diluted compounds (200×) was transferred to the wells containing 100 µL of the culture medium. Meanwhile the day 0 plate was measured by Cell TiterGlo as listed below.
d. Cells were incubated with the compounds for 72h at 5% CO₂, 37°C.

### Preparation of reagents:

The CellTiter-Glo buffer was thawed and equilibrated to room temperature prior to use. The lyophilized CellTiter-Glo substrate was equilibrated to room temperature prior to use. The appropriate volume of CellTiter-Glo buffer was transferred into the amber bottle containing CellTiter-Glo substrate to reconstitute the lyophilized enzyme/substrate mixture, thereby forming the CellTiter-Glo reagent. Mixing was carried out by gently vortexing, swirling or by inverting the contents to obtain a homogeneous solution, the CellTiter-Glo substrate going into solution easily in less than one minute.

### Assay measurement

The cell morphology was observed under an inverted microscope after corresponding treatment. The plate and its contents were equilibrated to room temperature for approximately 30 minutes, after which 100µL of CellTiter-Glo reagent was added to the assay plate by Multidrop Combi instrument and the contents mixed for 10 minutes on an orbital shaker to induce cell lysis. The plate was allowed to incubate at room temperature for 10 minutes to stabilize luminescent signal and the clear bottom pasted with white back seal and the luminescence recorded with Enspire with the settings: Luminescence, measurement time 0.1ms.

The results are shown in Table 6, the data being expressed as absolute IC₅₀ in µM.

**Table 6**

| **Compound ID** | HCC1954 | HCC38 | HCC1143 | MDA-MB-361 | T-47D | BT-474 |
|---|---|---|---|---|---|---|
| Dactolisib | 0.0145 | 0.0664 | 0.1449 | 0.0301 | 0.0565 | 0.1144 |
| Example 1 | 8.0414 | 5.5608 | 5.5554 | 9.0013 | 6.1875 | 7.7679 |
| Staurosporine | 0.0624 | 0.0015 | 0.0110 | 0.1178 | 0.2188 | 0.0766 |

The results show that the compound of Example 1 was potent against the cell lines *in vitro* and can therefore be expected to exhibit efficacy against breast cancer *in vivo.*

### Biological Example 8 - in vivo efficacy in leukaemia model

The anti-tumour efficacy of the compound of Example 1 was measured in human leukaemia KG-1 subcutaneous xenograft models in CB-17 SCID mice. The groups and treatments for the *in vivo* efficacy study are shown in Table 7 below.

**Table 7**

| # | Group | N | Dose level | Dose Route | Dose schedule |
|---|---|---|---|---|---|
| | | | (mg/kg/day) | | |
| 1 | Vehicle | 8 | - | oral | QD*21 |
| 2 | Ex 1, 25 | 8 | 25*2 | oral | BID*21 |
| 3 | Ex 1, 50 | 8 | 50 | oral | QD*21 |
| 4 | Ex 1, 100 | 8 | 100 | oral | QD*(1-21) |

Mice grouping was on day 0 (D0) and Example 1 25 BID*21 group was started on the evening of day 0 (D0), other groups were started on the morning of day 1 (D1). At the 21 days of the treatment, 8h and 24h post the last dose, 4 mice each from vehicle group, Example 1 25 mg/kg BID group and Example 1 50mg/kg group were subjected tumour collection.

Animals: Female mice (*Mus musculus)* strain: CB-17 SCID; 6-8 weeks old Body weight: 18-20 g; were obtained from Shanghai Lingchang BioTech Co., Ltd. Of the 59 obtained, 40 were grouped for efficacy study. The mice were kept in individual ventilation cages at constant temperature (20~26°C) and humidity (40-70%) with 5 animals in each cage. Cages were made of polycarbonate and the bedding material was corn cob, which was changed twice a week.: Animals had free access to irradiation sterilized dry granule food and to sterile drinking water during the entire study period.

Reagents: DMSO and PEG-400 were obtained from Sigma.

Formulation preparation:
Step 1: Firstly, a 100 mg / mL solution of Example 1 in DMSO was prepared. 0.24 mL DMSO was dispensed into a volume marked vial (5 ml), then 5mg of the compound of Example 1 was added and mixed until dissolved. Aliquots (~5mg) of Example 1 were the resulting solution and mixed until dissolved and until 100 mg/mL has been achieved (a total of 24 mg of compound will be required). The drug was dissolved moderately quickly and so allowed a reasonable time for preparation.
Step 2: (10mg/mL formulation): To the vial containing 100 mg/mL of the compound of Example 1 in DMSO solution prepared in step 1 was added to 2.0 mL of PEG400 and mixed until a clear solution is obtained. Additional PEG400 was added to 2.4 mL and mixed until homogeneous.

Cell Culture: The KG-1 (leukemia, acute myelogenous) cell was purchased from ATCC (ATCC^{®} CCL-246^{™}). The base medium for this cell line is ATCC-formulated Iscove's Modified Dulbecco's Medium, Catalog No. 30-2005. To make the complete growth medium, the following components were added to the base medium: fetal bovine serum to a final concentration of 20%. Cultures were then maintained by the addition of fresh medium or replacement of medium or alternatively, cultures were established by centrifugation with subsequent resuspension at 2 x 10⁵ viable cells/mL. Cell density between 2 x 10⁵ and 1 x 10⁶ viable cells/mL was maintained, while not being allowed to exceed 2 x 10⁶ cells/mL. Reference: https://www.atcc.org/Products/All/CCL-246.aspx#culturemethod

Tumour Inoculation: The mice were inoculated subcutaneously at the right flank with KG-1 tumour cells (5 x 10⁶ cells /mouse) in 0.2 mL mixture of base media with 50% Matrigel for tumour development.

All the procedures related to animal handling, care, and the treatment in this study were performed according to the guidelines approved by the Institutional Animal Care and Use Committee (IACUC) of Shanghai ChemPartner following the guidance of the Association for Assessment and Accreditation of Laboratory Animal Care (AAALAC). At the time of routine monitoring, the animals were checked for any effects of normal behavior such as mobility, food and water consumption (by visual checking only), body weight gain/loss (body weights were measured thrice weekly), eye/hair matting and any other abnormal effect. Death and observed clinical signs were recorded.

Tumour Measurements and the Endpoints: The major endpoint was tumour growth delay or cure. Tumour size was measured thrice weekly in two dimensions using a caliper, and the volume was expressed in mm³ using the formula: V = 0.5 a x b² where a and b were the long and short diameters of the tumour, respectively. Tumour size was measured in two dimensions using a caliper, and the volume was expressed in mm³ using the formula: V = 0.5 × a × b² where a and b are the long and short diameters respectively. The tumour size was then used for calculations TGI values. TGI was calculated according to the following equation: TGI (%) = (1- (TVTreatment/Dn - TVTreatment/D1)/ (TVControl/Dn- TVControl/D1)) × 100%.

Sample Collections: At the 21 days of the treatment, 8h and 24h post the last dose, 4 mice each from vehicle group, Example 1 25 mg/kg BID group and Example 1 50mg/kg group were subjected to tumour collection.

Statistical Analysis: Summary statistics, including mean and the standard error of the mean (SEM), were provided for the tumour volumes of each group at each time point. Statistical analyses of difference in tumour volumes among the groups were conducted on D21 after the last dose. Statistical analysis of difference in tumour volume and tumour weight among the groups was conducted. All data were analyzed using GraphPad Prism software. P< 0.05 was considered to be statistically significant. A two-way ANOVA combined with Bonferroni post-test was performed to compare tumour volumes among vehicle and all treatment groups. A one-way ANOVA combined with Dunnett post-test was performed to compare tumour weights among vehicle and all treatment groups.

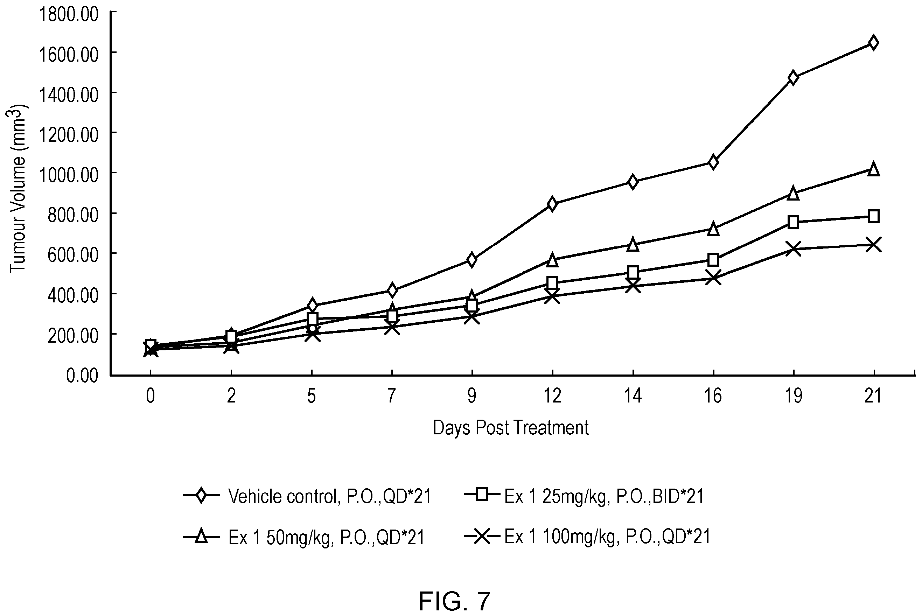

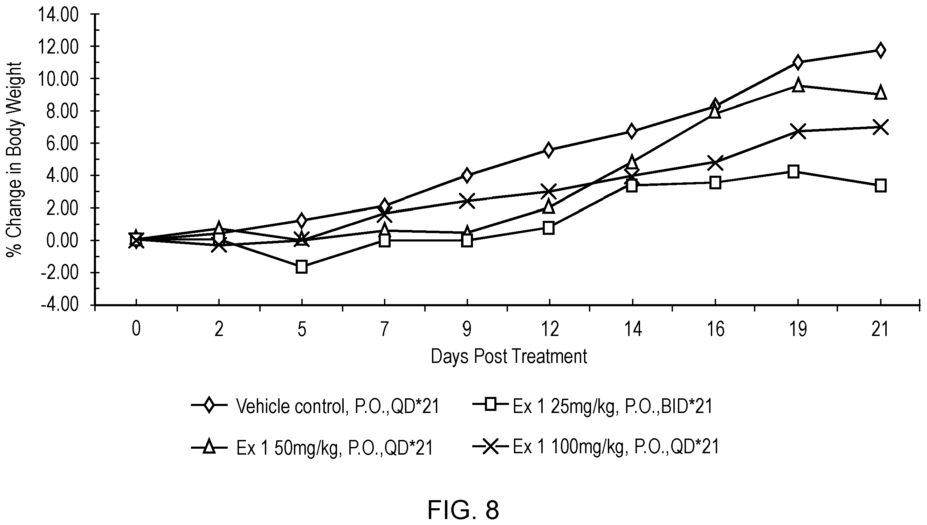

The results are shown in Figures 7 and 8, Figure 7 showing the tumour volume from 0 to 21 days post-treatment with the compound of Example 1 at 25, 50 and 100 mg/kg compared with control (vehicle) and Figure 8 showing % change in body weight from 0 to 21 days post-treatment with the compound of Example 1 at 25, 50 and 100 mg/kg compared with control (vehicle).

The results show that the compound is effective in this mouse model of leukaemia. It can therefore be expected to show *in vivo* efficacy against leukaemia in humans.

### Biological Example 9

This Biological Example demonstrates that the compound of Example 1 suppresses pathogenic Th1/Th17 CD4+ T cell responses while enhancing the generation of regulatory CD4+ T cells.

In chronic inflammatory diseases such as psoriasis and IBD, effector T cell subsets known as Th1 cells (which secrete IFNγ) and Th17 cells (which secrete IL-17A) are established drivers of pathogenesis, whereas iTreg cells (induced Regulatory T cells) are considered key mediators of immune suppression and resolution (Hu, P et al. Front Immunol. 2021 Dec 15;12:788940 and Nussbaum, L. et al. Br. J. Dermatol. 2021 Jan;184(1):14-24).

We examined the effects of inhibition of PIM1/2 kinases on the development of these subsets *in vitro* across a range of concentrations and determined that at an optimal concentration (12.5nM), the presence of the compound of Example 1 led to a significant suppression of 'damaging' pro-inflammatory Th1 and Th17 responses while enhancing the generation of immunoregulatory iTreg cells.

### Methods:

### CD4⁺ T_{H} cell culture and differentiation

Naïve CD4⁺ T cells were purified from mice spleens and lymph nodes using positive selection by magnetic beads (CD4 L3T4 Kit, Miltenyi Biotec, UK). The purified T cells were activated by plate-bound αCD3ε (1µg/ml; 2C11) and αCD28 (3µg/ml; 37.51) in the presence or absence of the compound of Example 1 or Cyclosporin A (Sigma Aldrich), and cultured for 72hrs under Th1 conditions (IL-12 [20ng/ml] + αIL-4 (11B1) [10µg/ml]), 72hrs under Th17 conditions (αIFNγ [10µg/ml] + αIL-4 [5µg/ml] + IL-6 [20ng/nl] + TGFβ(5ng/ml)), and 96hrs under iTreg conditions (TGFβ (5ng/ml)) at 37°C.

### ELISA

ELISA kits for mouse IFNγ and IL-17a were purchased from eBioscience (Thermo Fisher, UK) and performed in accordance to manufacturer's instructions using Corning^{®} High Binding ELISA plates (Merck, CLS9018). All ELISAs were analysed using a Synergy MX microplate reader (BioTek).

### Flow Cytometry

Intracellular protein expression was evaluated by first restimulating the cells with phorbol-12-myristate 13-acetate (PMA)(10ng/ml) (Sigma Aldrich), ionomycin (1µg/ml) (Sigma Aldrich) and Brefeldin A (5µg/ml) (eBioscience) for 4-6 hours at 37°C. A FOXP3 staining buffer set (eBiosciences/Thermo Fisher, UK) was used in accordance with manufacturer's instructions to fix and permeabilize cells to facilitate detection of intracellular cytokines (IFNγ and IL-17a) and transcription factor (FoxP3). Initial gating was performed on live cells using Live Dead fixable Aqua Dead Cell stain kit (Invitrogen). Fc block (93) and all fluorescence dye labelled antibodies used αCD4 (GK1.5), αIFNγ (XMG1.2), αIL-17a (17B7), αFOXP3 (NRRF-30; FJK-16S) were purchased from eBiosciences (Thermo Fisher, UK). Multi-parameter analysis was performed on an LSR/Fortessa (Becton Dickinson Biosciences (BD)) and analysed using FlowJo software (Tree Star).

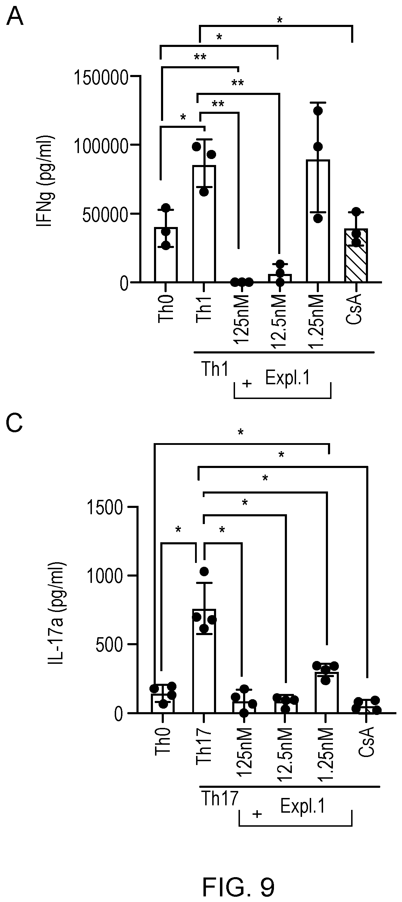

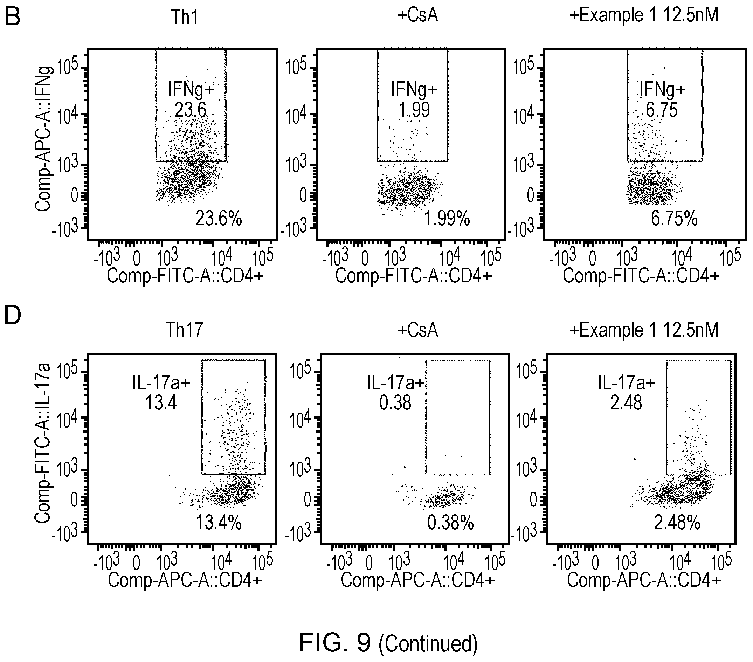

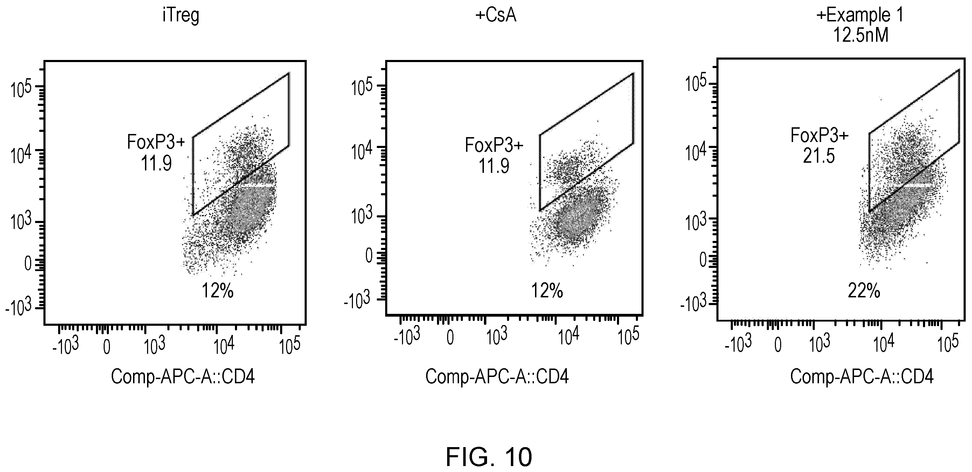

The results are shown in Figures 9 and 10. In particular, Figure 9 demonstrates that the compound of Example 1 inhibits the generation of Th1 and Th17 responses. Differentiation of Th1 cells *in vitro* as determined by the secretion of IFNγ is inhibited by the presence of the compound of Example 1 across a range of concentrations as determined by (A) ELISA and (B) FACS analysis of levels of IFNγ expression by CD4+ T cells. Differentiation of Th17 cells *in vitro* as determined by the secretion of IL-17A is inhibited by the presence of the compound of Example 1 at a range of concentrations as determined by (C) ELISA and (D) FACS analysis of levels of IL-17A expression by CD4+ T cells. Cyclosporin A (CsA), included as a control, established T cell immunosuppressant therapy. The data shown are representative of 2 independent experiments with similar results: statistical analysis used unpaired student's t test, where * represents P<0.05 and ** represents P<0.01.

Figure 10 further demonstrates that the compound of Example 1 promotes the generation of iTreg responses. Differentiation of iTreg cells *in vitro* as determined by the levels of intracellular expression of the Treg marker FoxP3 by FACS analysis.

Cyclosporin A (CsA) included as control established T cell immunosuppressant therapy. The data shown are representative of 2 independent experiments with similar results.

Furthermore, and in contrast to CsA, the presence of the compound of Example 1 (12.5nM) led to significantly increased expression of the transcription factor FoxP3, which confers regulatory/suppressive T cell function, when CD4+ T cells were differentiated under inducible Treg (iTreg) conditions in vitro (Figure 10).

Together these data indicate that the compound of Example 1 modulates the development of effector CD4+ T cell subsets towards a more anti-inflammatory profile.

### Biological Example 10 - in vitro potency against leukemia cell lines

The potency of the compound of Example 1 against a range of cell lines for leukemia was evaluated. Staurosporine (STS) (from Sigma, Cat. No. S4400-1MG, Lot. No. #115M4047V) and dactolisib (BEZ-235) were used as references.

*Materials:* RPMI1640 (from Invitrogen, Cat. No. 11875-093; Lot. No.1811359), IMDM (from Invitrogen, Cat. No. 12440-053; Lot. No.1806052), FBS (from Invitrogen, Cat. No. 10099-141, Lot. No. 1652792), Penicillin-Streptomycin solution (from Hyclone, Cat. No. SV30010, Lot. No. J160016), Glutamax (from Gibco, Cat. No. 35050-061; Lot. No. 1715705), DMSO (from Sigma, Cat. No. 276855-1L. Lot. No. #STBD8882V), 96-well plate, white wall with clear bottom, tissue culture treated (from Corning, Cat. No. CLS3903; Lot.No.16816002), 96 well, Square V-Bottom(from Costar, Cat.No.3960; Lot.No.04914002), CellTiter Glo assay kit (from Promega, Cat. No. G7571, Lot. No. 0000126342), staurosporine (from sigma, Cat. No. S4400-1MG, Lot. No. #115M4047V).

The cell lines are shown in Table 8. In all cases, the incubation time was 72 hours and the cell lines were grown as a suspension. The seeding density was per well in a 96 well plate.

**Table 8**

| **Cell line** | **Vendor** | **Cat#** | **Description** | **Complete medium** | **Seeding Density** |
|---|---|---|---|---|---|
| KG-1 | ATCC | CCL-246 | leukemia, acute myelogenous | IMDM+20%FBS | 10000 |
| MV-4-11 | ATCC | CRL-9591 | leukemia, biphenotypic B myelomonocytic | IMDM+10%FBS | 15000 |
| Kasumi-3 | ATCC | CRL-2725 | leukemia, acute myeloblastic leukemia | RPMI 1640 + 20% FBS | 30000 |
| JeKo-1 | ATCC | CRL-3006^{™} | lymphoma, mantle cell lymphoma | RPMI 1640+20%FBS | 20000 |
| Molm-13 | AddexBio | C0003003 | acute, myeloid, leukemia suspension | RPMI1640+20%FBS | 10000 |
| OCI-M1 | DSMZ | ACC-529 | leukemia, acute myeloid leukemia | IMDM+20%FBS | 10000 |
| HL-60 | ATCC | CCL-240 | leukemia, acute promyelocytic | IMDM+20%FBS | 15000 |

### Experimental Methods

The cell seeding methods, compound preparation, compound plate preparation, preparation of reagents, and assay measurement methods were the same as in Biological Example 7 above.

The results are shown in Table 9, the data being expressed as absolute IC₅₀ in µM.

**Table 9**

| **Compound ID** | KG-1 | MV-4-11 | Kasumi-3 | JeKo-1 | Molm-13 | OCI-M1 | HL-60 |
|---|---|---|---|---|---|---|---|
| Dactolisib | 0.0900 | 0.0365 | 0.1052 | 0.0678 | 0.0443 | 0.1249 | 0.4300 |
| Example 1 | 0.7152 | 0.9066 | 0.2322 | 6.5416 | 9.3421 | >10 | 8.7474 |
| Staurosporine | 0.0232 | 0.0003 | 0.0014 | 0.0107 | 0.0016 | 0.0484 | 0.0264 |

The results show that the compound of Example 1 was potent against the cell lines *in vitro* and can therefore be expected to exhibit efficacy against leukemia *in vivo.*

## Claims

1. A compound of formula I: 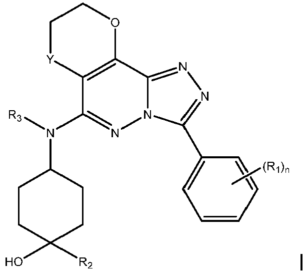 wherein:
Y is selected from the group consisting of C(Rₐ)(R_{b}) and NR_{c};
Rₐ, R_{b} and R_{c} are each independently selected from the group consisting of H and C₁₋₆ alkyl;
n is 1 to 3;
each R₁ is selected from the group consisting of halogen, C₁₋₆ alkyl, C₁₋₆ haloalkyl, C₁₋₆ alkoxy, and C₁₋₆ haloalkoxy;
R₂ is C₁₋₄ alkyl; and
R₃ is selected from the group consisting of H and C₁₋₃ alkyl;
or a pharmaceutically acceptable solvate or salt thereof.

2. A compound according to claim 1, wherein Y is selected from the group consisting of CH₂ and N(CH₃).

3. A compound according to claim 1 or claim 2, wherein n is 1 or 2.

4. A compound according to any one of claims 1 to 3, wherein each R₁ is selected from the group consisting of F, CF₃, and OCF₃.

5. A compound according to any one of claims 1 to 4, wherein n is 1 and R₁ is OCF₃.

6. A compound according to any one of claims 1 to 4, wherein n is 2, one R₁ is F and the other is CF₃.

7. A compound according to any one of claims 1 to 5, wherein R₂ is CH₃.

8. A compound according to any one of claims 1 to 5, wherein R₃ is H.

9. A compound according to claim 1, of formula: 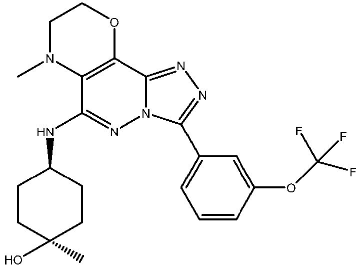 or a pharmaceutically acceptable solvate or salt thereof.

10. A compound according to claim 1, of formula: 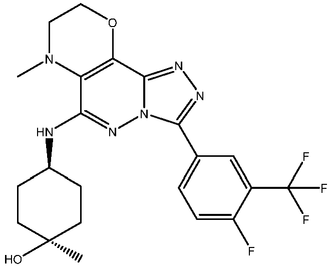 or a pharmaceutically acceptable solvate or salt thereof.

11. A compound according to claim 1, of formula: 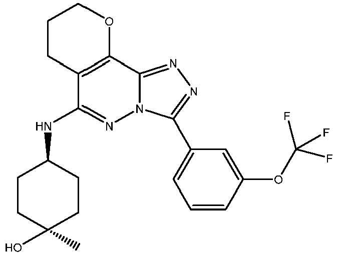 or a pharmaceutically acceptable solvate or salt thereof.

12. A pharmaceutical formulation including a compound of formula I, as defined in any one of Claims 1 to 11, or a pharmaceutically acceptable solvate or salt thereof, in admixture with a pharmaceutically acceptable adjuvant, diluent or carrier.

13. A compound of formula I as defined in any one of Claims 1 to 11, or a pharmaceutically acceptable solvate or salt thereof, for use as a medicament.

14. A compound of formula I as defined in any one of Claims 1 to 11, or a pharmaceutically acceptable solvate or salt thereof, for use in the treatment of a disease selected from the group consisting of cancer, an immune disorder, a cardiovascular disease, a viral infection, inflammation, a metabolism/endocrine function disorder, a neurological disorder, an obstructive airways disease, an allergic disease, an inflammatory disease, immunosuppression, a disorder commonly connected with organ transplantation, an AIDS-related disease, benign prostate hyperplasia, familial adenomatosis, polyposis, neuro-fibromatosis, psoriasis, a bone disorder, atherosclerosis, vascular smooth cell proliferation associated with atherosclerosis, pulmonary fibrosis, arthritis, glomerulonephritis and post-surgical stenosis, restenosis, stroke, diabetes, hepatomegaly, Alzheimer's disease, cystic fibrosis, a hormone-related disease, an immunodeficiency disorder, a destructive bone disorder, an infectious disease, a condition associated with cell death, thrombin-induced platelet aggregation, chronic myelogenous leukaemia, liver disease, a pathologic immune condition involving T cell activation, CNS disorders, and other associated diseases.

15. A combination comprising:
(A) a compound of formula I as defined in any one of Claims 1 to 11, or a pharmaceutically-acceptable solvate or salt thereof; and
(B) another therapeutic agent.

## Patentansprüche

1. Verbindung der Formel I: 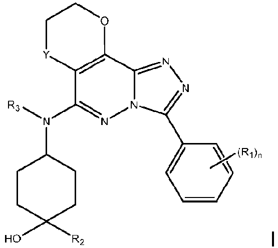 wobei:
Y aus der Gruppe bestehend aus C(Rₐ)(R_{b}) und NR_{c} ausgewählt ist;
Rₐ, R_{b} und R_{c} jeweils unabhängig aus der Gruppe bestehend aus H und C₁₋₆-Alkyl ausgewählt sind;
n für 1 bis 3 steht;
R₁ jeweils aus der Gruppe bestehend aus Halogen, C₁₋₆-Alkyl, C₁₋₆-Halogenalkyl, C₁₋₆-Alkoxy und C₁₋₆-Halogenalkoxy ausgewählt ist;
R₂ für C₁₋₄-Alkyl steht und
R₃ aus der Gruppe bestehend aus H und C₁₋₃-Alkyl ausgewählt ist;
oder ein pharmazeutisch unbedenkliches Solvat oder Salz davon.

2. Verbindung nach Anspruch 1, wobei Y aus der Gruppe bestehend aus CH₂ und N(CH₃) ausgewählt ist.

3. Verbindung nach Anspruch 1 oder Anspruch 2, wobei n für 1 oder 2 steht.

4. Verbindung nach einem der Ansprüche 1 bis 3, wobei R₁ jeweils aus der Gruppe bestehend aus F, CF₃ und OCF₃ ausgewählt ist.

5. Verbindung nach einem der Ansprüche 1 bis 4, wobei n für 1 steht und R₁ für OCF₃ steht.

6. Verbindung nach einem der Ansprüche 1 bis 4, wobei n für 2 steht, ein R₁ für F steht und das andere für CF₃ steht.

7. Verbindung nach einem der Ansprüche 1 bis 5, wobei R₂ für CH₃ steht.

8. Verbindung nach einem der Ansprüche 1 bis 5, wobei R₃ für H steht.

9. Verbindung nach Anspruch 1 mit der Formel: 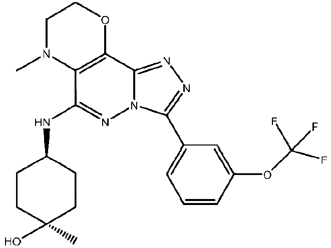 oder ein pharmazeutisch unbedenkliches Solvat oder Salz davon.

10. Verbindung nach Anspruch 1 mit der Formel: 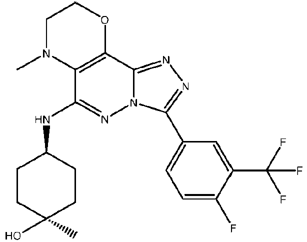 oder ein pharmazeutisch unbedenkliches Solvat oder Salz davon.

11. Verbindung nach Anspruch 1 mit der Formel: 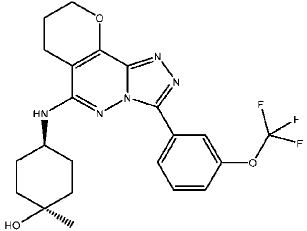 oder ein pharmazeutisch unbedenkliches Solvat oder Salz davon.

12. Pharmazeutische Formulierung, enthaltend eine Verbindung der Formel I gemäß einem der Ansprüche 1 bis 11 oder ein pharmazeutisch unbedenkliches Solvat oder Salz davon in Abmischung mit einem pharmazeutisch unbedenklichen Adjuvans, Verdünnungsmittel oder Träger.

13. Verbindung der Formel I gemäß einem der Ansprüche 1 bis 11 oder pharmazeutisch unbedenkliches Solvat oder Salz davon zur Verwendung als Medikament.

14. Verbindung der Formel I gemäß einem der Ansprüche 1 bis 11 oder pharmazeutisch unbedenkliches Solvat oder Salz davon zur Verwendung bei der Behandlung einer Erkrankung, die aus der Gruppe bestehend aus Krebs, einer Immunstörung, einer Herz-Kreislauf-Erkrankung, einer Virusinfektion, Entzündung, einer Stoffwechsel/Endokrinum-Funktionsstörung, einer neurologischen Störung, einer obstruktiven Atemwegserkrankung, einer allergischen Erkrankung, einer entzündlichen Erkrankung, Immunsuppression, einer Störung, die gemeinhin mit Organtransplantation verbunden ist, einer mit AIDS in Zusammenhang stehenden Erkrankung, benigner Prostatahyperplasie, familiärer Adenomatose, Polypose, Neurofibromatose, Psoriasis, einer Knochenstörung, Atherosklerose, mit Atherosklerose assoziierter Proliferation von glatten Gefäßzellen, Lungenfibrose, Arthritis, Glomerulonephritis und postoperativer Stenose, Restenose, Schlaganfall, Diabetes, Lebervergrößerung, Alzheimer-Krankheit, zystischer Fibrose, einer mit Hormonen in Zusammenhang stehenden Erkrankung, einer Immunmangelerkrankung, einer destruktiven Knochenstörung, einer Infektionserkrankung, einem mit Zelltod assoziierten Leiden, thrombininduzierter Thrombozytenaggregation, chronischer myeloischer Leukämie, Lebererkrankung, einem pathologischen Immunleiden mit T-Zell-Aktivierung, ZNS-Störungen und anderen assoziierten Erkrankungen ausgewählt ist.

15. Kombination, umfassend:
(A) eine Verbindung der Formel I gemäß einem der Ansprüche 1 bis 11 oder ein pharmazeutisch unbedenkliches Solvat oder Salz davon und
(B) ein anderes Therapeutikum.

## Revendications

1. Composé de formule I : 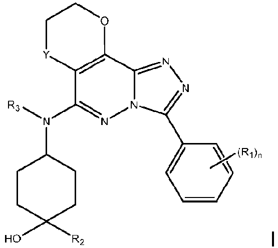 dans laquelle :
Y est choisi dans le groupe constitué par C(Rₐ)(R_{b}) et NR_{c} ;
Rₐ, R_{b} et R_{c} sont chacun indépendamment choisis dans le groupe constitué par H et C₁₋₆ alkyle ;
n est 1 à 3 ;
chaque R₁ est choisi dans le groupe constitué par halogène, C₁₋₆ alkyle, C₁₋₆ halogénoalkyle, C₁₋₆ alcoxy, et C₁₋₆ halogénoalcoxy ;
R₂ est C₁₋₄ alkyle ; et
R₃ est choisi dans le groupe constitué par H et C₁₋₃ alkyle ;
ou solvate ou sel pharmaceutiquement acceptable correspondant.

2. Composé selon la revendication 1, dans lequel Y est choisi dans le groupe constitué par CH₂ et N(CH₃).

3. Composé selon la revendication 1 ou la revendication 2, dans lequel n est 1 ou 2.

4. Composé selon l'une quelconque des revendications 1 à 3, dans lequel chaque R₁ est choisi dans le groupe constitué par F, CF₃, et OCF₃.

5. Composé selon l'une quelconque des revendications 1 à 4, dans lequel n est 1 et R₁ est OCF₃.

6. Composé selon l'une quelconque des revendications 1 à 4, dans lequel n est 2, un R₁ est F et l'autre est CF₃.

7. Composé selon l'une quelconque des revendications 1 à 5, dans lequel R₂ est CH₃.

8. Composé selon l'une quelconque des revendications 1 à 5, dans lequel R₃ est H.

9. Composé selon la revendication 1, de formule : 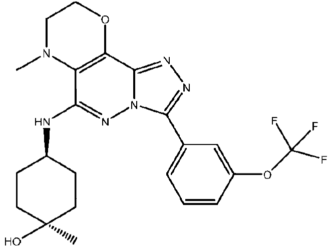 ou solvate ou sel pharmaceutiquement acceptable correspondant.

10. Composé selon la revendication 1, de formule : 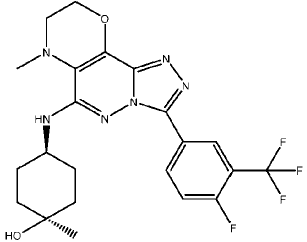 ou solvate ou sel pharmaceutiquement acceptable correspondant.

11. Composé selon la revendication 1, de formule : 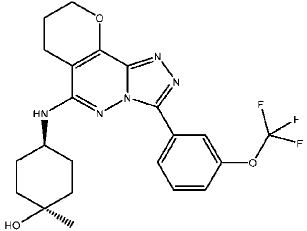 ou solvate ou sel pharmaceutiquement acceptable correspondant.

12. Formulation pharmaceutique comprenant un composé de formule I, tel que défini dans l'une quelconque des revendications 1 à 11, ou un solvate ou sel pharmaceutiquement acceptable de celui-ci, en mélange avec un adjuvant, diluant ou support pharmaceutiquement acceptable.

13. Composé de formule I tel que défini dans l'une quelconque des revendications 1 à 11, ou solvate ou sel pharmaceutiquement acceptable de celui-ci, destiné à être utilisé comme médicament.

14. Composé de formule I tel que défini dans l'une quelconque des revendications 1 à 11, ou solvate ou sel pharmaceutiquement acceptable correspondant, pour utilisation dans le traitement d'une maladie choisie dans le groupe constitué par un cancer, un trouble immunitaire, une maladie cardiovasculaire, une infection virale, une inflammation, un trouble du métabolisme/de la fonction endocrinienne, un trouble neurologique, une maladie obstructive des voies respiratoires, une maladie allergique, une maladie inflammatoire, une immunosuppression, un trouble fréquemment associé à la transplantation d'organes, une maladie liée au SIDA, une hyperplasie bénigne de la prostate, une adénomatose familiale, une polypose, une neurofibromatose, le psoriasis, un trouble osseux, l'athérosclérose, la prolifération des cellules lisses vasculaires associée à l'athérosclérose, la fibrose pulmonaire, l'arthrite, la glomérulonéphrite et la sténose post-chirurgicale, la resténose, un accident vasculaire cérébral, le diabète, l'hépatomégalie, la maladie d'Alzheimer, la mucoviscidose, une maladie liée aux hormones, un trouble de l'immunodéficience, un trouble osseux destructeur, une maladie infectieuse, une affection associée à la mort cellulaire, l'agrégation plaquettaire induite par la thrombine, la leucémie myéloïde chronique, une maladie du foie, une affection immunitaire pathologique impliquant l'activation des lymphocytes T, des troubles du SNC et d'autres maladies associées.

15. Combinaison comprenant :
(A) un composé de formule I tel que défini selon l'une quelconque des revendications 1 à 11, ou un solvate ou sel pharmaceutiquement acceptable de celui-ci ; et
(B) un autre agent thérapeutique.
